# EUROPÄISCHE PATENTSCHRIFT

(11) **EP 3 962 891 B1**
(45) Veröffentlichungstag und Bekanntmachung des Hinweises auf die Patenterteilung: **29.03.2023**
(21) Anmeldenummer: 20725626.4
(22) Anmeldetag: 27.04.2020
(51) Int. Cl.: C07C 273/02, C07C 231/10, C07C 233/03, C07C 273/10, C07F 15/00

(54) **VERFAHREN ZUR KATALYTISCHEN HERSTELLUNG VON HARNSTOFF**
METHOD FOR CATALYTICALLY PRODUCING UREA
PROCÉDÉ DE PRODUCTION CATALYTIQUE D'URÉE

(30) Priorität: 29.04.2019 DE 102019111058
(43) Veröffentlichungstag der Anmeldung: 09.03.2022
(73) Patentinhaber: thyssenkrupp Industrial Solutions AG, 45143 Essen (DE); thyssenkrupp AG, 45143 Essen (DE)
(72) Erfinder: MAKHYNYA, Yevgeny, 45481 Mülheim an der Ruhr (DE); EL HAWARY, Tarek, 59439 Holzwickede (DE); GLOTZBACH, Christoph, 32052 Herford (DE); TENHUMBERG, Nils, 44388 Dortmund (DE); LEITNER, Walter, 52074 Aachen (DE); KLANKERMAYER, Jürgen, 45259 Essen (DE); SCHUMACHER, Hannah, 52064 Aachen (DE)
(74) Vertreter: thyssenkrupp Intellectual Property GmbH
(86) Internationale Anmeldenummer: PCT/EP2020/061614
(87) Internationale Veröffentlichungsnummer: WO 2020/221690

(56) Entgegenhaltungen:
- WO-A1-2013/014160
- US-A- 2 083 010
- US-A1- 2012 071 690
- FRANCESCO BARZAGLI ET AL: "From greenhouse gas to feedstock: formation of ammonium carbamate from CO2 and NH3 in organic solvents and its catalytic conversion into urea under mild conditions", GREEN CHEMISTRY, Bd. 13, Nr. 5, 1. Januar 2011 (2011-01-01) , Seite 1267, XP55709627, GB ISSN: 1463-9262, DOI: 10.1039/c0gc00674b in der Anmeldung erwähnt

## Beschreibung

Die Erfindung betrifft ein Verfahren zur katalytischen Herstellung von Harnstoff.

Harnstoff, das Diamid der Kohlensäure, ist eine der wichtigsten Massenchemikalien und wird überwiegend als Düngemittel eingesetzt. Als solches besitzt er einen hohen Stickstoffgehalt (46 Gew.-%). Er wird durch das im Boden häufig vorkommende, von Mikroorganismen produzierte Enzym Urease leicht hydrolysiert, wodurch Ammoniak und COz freigesetzt werden.

Darüber hinaus ist Harnstoff ein wichtiger Baustein für organische Produkte, wie z.B. Melamin, und ein Rohstoff für Kunstharze und Fasern. Er wird als Zusatzstoff für Viehfutter verwendet und bei der Produktion von Pharmazeutika und Sprengstoffen sowie in der Textilindustrie eingesetzt. In den letzten Jahrzehnten hat Harnstoff auch als Reduktionsmittel für die NOx-Reduktion von Dieselabgasen an Bedeutung gewonnen.

Die großtechnische Herstellung von Harnstoff beruht derzeit praktisch ausschließlich auf der Hochdrucksynthese von Ammoniak (NH₃) und Kohlendioxid (CO₂) bei ca. 150 bar und ca. 180 °C. Beide Einsatzstoffe kommen in der Regel aus einer Ammoniak-Anlage, die meist in enger Nachbarschaft zu einer Harnstoff-Anlage steht. Ammoniak wird großtechnisch aus Stickstoff und Wasserstoff gewonnen, wobei für die Einsatzstoffe ein Synthesegas eingesetzt wird, aus dem störende Stoffe wie Schwefelverbindungen oder Kohlendioxid entfernt werden.

Ein bedeutsames Konzept für die Harnstoffsynthese beruht dabei auf der sogenannten Carbamatroute. Hierbei wird das als Ausgangsmaterial erforderliche Kohlendioxid durch Abtrennung aus einem für die Ammoniak-Synthese produzierten Synthesegas gewonnen. Bei dem Prozess wird das im Vorfeld abgetrennte COz mit flüssigem Ammoniak in Verbindung gebracht. Dabei wird im ersten Syntheseschritt vorwiegend Ammoniumcarbamat synthetisiert. Während des Reaktionsverlaufs wird auch Harnstoff in kleinen Mengen gebildet, so dass eine komplexe Mischung aus Ammoniak, COz, Harnstoff, Ammoniumcarbamat, Ammoniumhydrogencarbonat und Wasser entsteht. Dies geschieht in einem Apparat, der als Carbamatkondensor bezeichnet wird. Das Reaktionsgemisch verlässt den Carbamatkondensor in Richtung Harnstoffreaktor, wo die eigentliche Umsetzung zum Harnstoff stattfindet.

Weil das Carbamat ein hochkorrosives Medium ist, vor allem bei hohen Temperaturen und Drücken, ist an vielen Stellen des Verfahrens ein spezieller, korrosionsbeständiger Stahl erforderlich. Ein dafür geeigneter Spezialstahl ist im Handel unter der Bezeichnung Safurex^{®} erhältlich. Genutzt wird der Safurex-Stahl als Werkstoff u.a. für die inneren Beschichtungen der Apparate und für die Rohrleitungen. Die Anschaffung bzw. Nutzung dieses Stahls ist äußerst kostspielig und erhöht die Kapitalkosten der Anlage enorm. Nicht nur der Stahl, sondern auch der Prozess mit hohem Druck und Temperatur stellt eine große Herausforderung für die Apparate des Hochdruckkreislaufes dar, was sich letztendlich in den Anschaffungskosten für diese Apparate niederschlägt. Diese Nachteile können durch die hier vorliegende Erfindung überwunden werden.

Substituierte Harnstoffderivate können katalytisch über verschiedene Routen hergestellt werden, wobei CO und COz oder andere Carbonylierungsmittel eingesetzt werden. Die Synthese substituierter Harnstoffderivate mittels COz wird z.B. in P. Munshi, et al., Tetrahedron Lett. 2003, 44, 2725-2727, beschrieben. Über die Synthese mit anderen Carbonylierungsmitteln wird z.B. in A. Basha, Tetrahedron Lett. 1988, 29, 2525-2526, berichtet.

Gegenüber dem Einbau von Aminen für substituierte Harnstoffe ergeben sich beim Einsatz von Ammoniak zur Herstellung von Harnstoff zusätzliche Herausforderungen, da Ammoniak drei potentiell aktive Wasserstoffe und eine signifikant andere Basizität aufweist. Es gibt daher nur relativ wenige Veröffentlichungen, die über eine katalytische Synthese von Harnstoff berichten, siehe z.B. F. Barzagli et al., Green Chem. 2011, 13, 1267-1274, M. M. Taqui Khan et al., J. Mol. Catal. 1988, 48, 25-27, D. C. Butler et al., Inorg. Chem. Commun. 1999, 2, 305-307.

Die Erfinder haben die Überlegung angestellt, ein Verfahren zur katalytischen Synthese von Harnstoff auf Basis von Formamid als Ausgangsmaterial bereitzustellen, um die vorstehend beschriebenen Nachteile der konventionellen Verfahren zu überwinden.

Die großtechnische Herstellung von Formamid, auch Methanamid oder Ameisensäureamid genannt, beruht derzeit praktisch ausschließlich auf zwei synthetischen Routen, beide auf Basis von Kohlenmonoxid als C1-Ausgangsstoff und beide mit Natriummethylat als Katalysator. Bei der ersten Route handelt es sich um die direkte Carbonylierung von Ammoniak (NH₃) mit Kohlenmonoxid (CO) (GI. 1).

CO + NH₃ → HCONH₂ (GI. 1)

Die zweite Route beinhaltet die Reaktion von Kohlenmonoxid mit Methanol mit Natriummethylat als Katalysator zum Methylformiat mit nachfolgender Ammonolyse (GI. 2-3). Freigesetztes Methanol wird dabei rezykliert.

CO + CH₃OH → HCOOCH₃ (GI. 2)

HCOOCH₃ + NH₃ → HCONH₂ + CH₃OH (GI. 3)

Für beide Routen wird CO eingesetzt. Das CO ist einerseits ein sehr wertvolles, andererseits auch ein sehr toxisches Edukt, dessen Herstellung zudem recht aufwändig ist.

Eine Alternative zur Nutzung von CO stellt die Nutzung von Kohlendioxid (CO₂) dar. Die Nutzung von COz für die C1-Chemie ist dabei insbesondere aus sicherheitsrelevanten und wirtschaftlichen Gründen sehr attraktiv. Das COz ist eines der Produkte aus dem Dampfreformierungsprozess, welches aus dem Prozessgas in reinem oder fast reinem Zustand abgetrennt wird. Es kann sowohl aus dem Rauchgas als auch aus dem Reformergas in chemisch reinem Zustand isoliert werden.

Das Problem bei der stofflichen Nutzung von COz liegt jedoch an der thermodynamischen Stabilität von COz. Das Molekül verhält sich in den meisten chemischen Reaktionen inert. In seltenen Fällen gelingt jedoch die Aktivierung von COz mit Hilfe von speziellen Katalysatoren.

Die WO 2013/014160 A1 beschreibt die Bildung von Formamid aus Ammoniak, Kohlendioxid und Wasserstoff in Methanol unter Verwendung eines Katalysators aus 1 % Gold auf TiO₂ und die Bildung von Methylformiat aus Kohlendioxid und Wasserstoff in Methanol unter Verwendung eines Katalysators aus 1 % Gold auf TiOz oder Al₂O₃.

Die US 2012/071690 A1 beschreibt die Herstellung von Formamiden über ein Ammoniumformiat-Zwischenprodukt. Das Ammoniumformiat-Zwischenprodukt wird aus Kohlendioxid und Wasserstoff und einem tertiären Amin mit Hilfe eines Katalysators, bevorzugt enthaltend Ruthenium, Rhodium, Palladium, Osmium und/oder Iridium, gebildet, welches ein Addukt von Ameisensäure und dem tertiären Amin darstellt. Das Addukt wird anschließend abgetrennt und mit Ammoniak oder Aminen in das Formamid überführt.

In der DE 102012019441 A1 wird die Herstellung von Formamid mit Hilfe von Iridium-Katalysatoren aus den Edukten Methanol, Ammoniak, Kohlendioxid und Wasserstoff beschrieben.

Vaska et al. beschreiben in dem DTIC Document AD-A199 861 von 1988 die Herstellung von Formamid aus Ammoniak, Kohlendioxid und Wasserstoff mit Hilfe des Katalysators [Ir(Cl)(CO)(Ph₃P)₂].

Diese aus dem Stand der Technik bekannten Verfahren zur Herstellung von Formamid über die katalytische Aktivierung von COz haben den Nachteil, dass die Ausbeuten an Formamid relativ gering sind. Es findet zudem eine schnelle Deaktivierung der verwendeten Katalysatoren statt. Des Weiteren kann eine Reaktionsdauer von mehreren Tagen erforderlich sein.

Ammoniak ist das gängige Ausgangsmaterial bei der Synthese von Harnstoff. Ferner ist COz ein gut verfügbarer Einsatzstoff. Bei der Suche nach einer katalytischen Route zur Synthese von Harnstoff auf Basis von COz wurde als Ausgangspunkt ein zweistufiges Verfahren über Formamid als Zwischenprodukt ins Auge gefasst, wie in Schema 1 dargestellt:

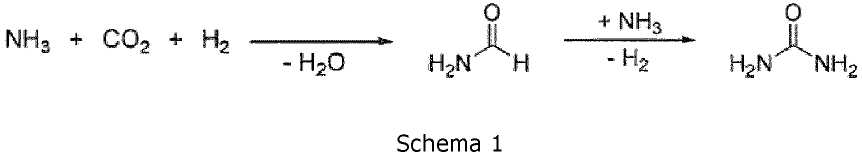

Während die Synthesen von substituiertem Harnstoff aus Formamiden in der Literatur beschrieben werden, siehe z.B. S. Kotachi, Y. Tsuji, T. Kondo, Y. Watanabe, J. Chem. Soc., Chem. Commun. 1990, 549-550, stellt die Bildung von Harnstoff aus der Umsetzung von Formamid bevorzugt mit Ammoniak eine neue und herausfordernde C-N-Bindungsbildung dar. Die Probleme des Einsatzes von CO₂ aufgrund seiner thermodynamischen Stabilität wurden vorstehend diskutiert.

Zusammenfassend ist festzustellen, dass bisherige Lösungen zur Synthese von Harnstoff nicht in allen Belangen zufriedenstellend sind. Es besteht daher ein Bedarf nach einer alternativen Harnstoffsynthese, bei der die Nachteile der konventionellen Harnstoff-Synthesen vermieden werden. Die Einsatzstoffe sollen weiterhin, aber nicht zwingend aus der Ammoniaksynthese stammen.

Der Erfindung liegt die Aufgabe zugrunde, ein für den großtechnischen Einsatz geeignetes alternatives Verfahren zur Herstellung von Harnstoff, vorzugsweise auf Basis von aus der Ammoniakanlage stammenden Einsatzstoffen, bereitzustellen, bei dem die Anforderungen an die Anlagen z.B. hinsichtlich Aggressivität gebildeter Stoffe sowie Druck- und Temperaturbedingungen verringert werden können. Insbesondere soll das Verfahren gut in eine übliche Anlage zur Ammoniaksynthese integriert werden können, d.h. die Einsatzstoffe für die Harnstoffsynthese sollen aus den Prozessströmen einer Ammoniakanlage entnommen werden können und allenfalls geringe Modifikationen bei dem Prozess der Ammoniaksynthese erfordern. Hauptmotiv ist ein möglichst minimaler Eingriff in die existierende Verfahrensgestaltung der Ammoniak-Anlage.

Diese Aufgabe wird erfindungsgemäß durch ein Verfahren gemäß Patentanspruch 1 gelöst.

Insbesondere wird die Aufgabe durch ein Verfahren zur Herstellung von Harnstoff gelöst, welches umfasst:
a) Herstellen von Formamid auf Basis von Kohlendioxid, Wasserstoff und Ammoniak, wobei Methylformiat oder Ammoniumformiat als Zwischenprodukt in einer katalytischen Reaktion gebildet wird, und
b) Herstellen von Harnstoff durch Umsetzung des gebildeten Formamids oder des gebildeten Formamids mit Ammoniak in Anwesenheit eines Katalysators,
wobei die Quelle für Kohlendioxid eine mit chemisch und/oder physikalisch gebundenem Kohlendioxid beladene Flüssigkeit, ausgewählt aus einer Methanolphase oder einer wässrigen Ammoniaklösung, ist, die erhalten wird durch Gaswäsche eines Synthesegases zur Entfernung von CO₂ unter Verwendung einer Waschflüssigkeit, wobei
a1) die Waschflüssigkeit eine Methanolphase ist oder das COz aus der mit chemisch und/oder physikalisch gebundenem Kohlendioxid beladene Waschflüssigkeit desorbiert und in eine Methanolphase absorbiert wird, um eine mit COz beladene Methanolphase zu erhalten, und
   die mit COz beladene Methanolphase als COz enthaltender Strom mit einem Wasserstoff enthaltenden Strom in Anwesenheit eines Katalysators unter Bildung von Methylformiat umgesetzt wird und das gebildete Methylformiat mit einem Ammoniak enthaltenden Strom unter Bildung von Formamid umgesetzt wird oder
a2) die Waschflüssigkeit eine wässrige Ammoniaklösung ist, so dass COz zumindest teilweise in Form von Carbonaten in der Waschflüssigkeit gebunden wird, und diese mit chemisch und/oder physikalisch gebundenem CO₂ beladene Waschflüssigkeit als COz enthaltender Strom mit einem Wasserstoff enthaltenden Strom in Anwesenheit von einem Katalysator und gegebenenfalls organischem Lösungsmittel unter Bildung von Ammoniumformiat oder unter Bildung von Ammoniumformiat und Formamid umgesetzt wird und das gebildete Ammoniumformiat durch Wärmebehandlung in Formamid überführt wird.

Ein wesentlicher Punkt der Erfindung liegt darin, dass das erfindungsgemäße Verfahren zur Herstellung von Harnstoff so konzipiert ist, dass Prozessströme der Ammoniaksynthese für die Einsatzstoffe genutzt werden können, wobei insbesondere der in dem Synthesegas enthaltene Wasserstoff, der z.B. in der Dampfreformierung erzeugt wird, ohne Verluste nutzbar gemacht werden kann, und dazu nur minimal in den Ammoniak-Prozess eingegriffen werden muss.

Bezüglich der hier vorgeschlagenen Lösung ist hervorzuheben, dass die Edukte bzw. Quellen der dreistufigen Harnstoff-Synthese, nämlich COz, H₂ und NH₃, in dem herkömmlichen Prozesskonzept einer Ammoniak-Anlage bereits zur Verfügung stehen und nur geringfügige Eingriffe in die Ammoniak-Synthese notwendig sind. Im Gegensatz dazu würde eine klassische Formamid-Synthese auf CO-Basis eine komplette Neuplanung des Ammoniak-Prozesses mit sich bringen. Ein weiterer wichtiger Punkt ist die Tatsache, dass der Wasserstoff während der Formamid-Synthese nicht verloren geht und nach der Harnstoffsynthese für die Ammoniak-Synthese wieder verfügbar gemacht werden kann, da der Wasserstoff bei der Umsetzung von Formamid oder von Formamid und Ammoniak zu Harnstoff wieder freigesetzt wird.

Weitere Ausführungsformen der Erfindung ergeben sich aus den Unteransprüchen. Im Folgenden wird die Erfindung im Einzelnen erläutert.

Das erfindungsgemäße Verfahren zur Herstellung von Harnstoff umfasst:
a) das Herstellen von Formamid auf Basis von Kohlendioxid, Wasserstoff und Ammoniak, wobei Methylformiat oder Ammoniumformiat als Zwischenprodukt in einer katalytischen Reaktion gebildet wird, und
b) das Herstellen von Harnstoff durch Umsetzung des gebildeten Formamids oder des gebildeten Formamids mit Ammoniak in Anwesenheit eines Katalysators,
wobei die Quelle für Kohlendioxid und gegebenenfalls Ammoniak eine mit chemisch und/oder physikalisch gebundenem Kohlendioxid beladene Flüssigkeit ist, die aus einer Methanolphase oder einer wässrigen Ammoniaklösung ausgewählt ist, und die direkt oder indirekt durch Gaswäsche eines Synthesegases zur Entfernung von CO₂ unter Verwendung der Waschflüssigkeit erhalten wird.

Das erfindungsgemäße Verfahren eignet sich insbesondere für die großtechnische Herstellung von Harnstoff. Das erfindungsgemäße Verfahren ist vorzugsweise ein kontinuierliches Verfahren.

Als Einsatzstoffe bzw. Quellen für das erfindungsgemäße Verfahren werden insbesondere Kohlendioxid, Wasserstoff und Ammoniak verwendet, die vorzugsweise aus einem Prozess zur Ammoniaksynthese stammen.

Als Quelle für Kohlendioxid und gegebenenfalls Ammoniak wird eine mit chemisch und/oder physikalisch gebundenem Kohlendioxid beladene Waschflüssigkeit eingesetzt, die durch Gaswäsche eines Synthesegases zur Entfernung von CO₂ unter Verwendung der Waschflüssigkeit erhalten wird.

Als Synthesegas dient bevorzugt ein aus der Dampfreformierung gasförmiger und/oder flüssiger Kohlenwasserstoffe und anschließender Wassergas-Shift-Reaktion erhaltenes Synthesegas, wie es z.B. für die Ammoniaksynthese eingesetzt wird. Das Synthesegas ist bevorzugt ein Synthesegas, welches für die Ammoniaksynthese erzeugt wird. Bis zur CO₂-Wäsche verläuft der Prozess zur Bildung des Synthesegases vorzugsweise identisch zu der klassischen Ammoniak-Synthese, bevorzugt über die Dampfreformierung, einschließlich Sekundärreformer, und anschließender Wassergas-Shift-Reaktion, die in der Regel zweistufig als Hoch- und Niedertemperatur-CO-Shift ausgeführt wird.

Im Einzelnen wird bei der Dampfreformierung ein kohlenstoffhaltiges Material, wie z.B. Erdgas oder ein anderer Kohlenwasserstoff, welcher gegebenenfalls vorgereinigt wird, in einem Primärreformer mit Wasserdampf umgesetzt, wobei das kohlenstoffhaltige Material weitgehend in CO, COz und Wasserstoff umgewandelt wird. In einem nachgeschalteten sogenannten Sekundärreformer, der hier, falls eingesetzt, als zur Dampfreformierung zugehörig angesehen wird, kann durch Zugabe von Prozessluft noch restliches kohlenstoffhaltiges Material umgesetzt werden, wobei durch die Prozessluft auch Stickstoff eingebracht wird. Hierüber kann auch das gewünschte H_{2/}N₂-Verhältnis eingestellt werden.

Bei einer anschließenden Wassergas-Shift-Reaktion wird Kohlenmonoxid und Wasserdampf in Kohlendioxid und Wasserstoff überführt. Die Wassergas-Shift-Reaktion wird häufig zweistufig als Hochtemperatur- und Niedertemperatur-Shiftstufe ausgeführt. Die Hauptkomponenten im erhaltenen Synthesegas sind Wasserstoff, Stickstoff und Kohlendioxid. Als Verunreinigungen können z.B. noch Kohlenmonoxid, Argon und geringe Mengen an Kohlenwasserstoffen enthalten sein.

Geeignete Verfahren zur Erzeugung eines solchen Synthesegases sind einem Fachmann bekannt und es kann diesbezüglich beispielsweise vollumfänglich verwiesen werden auf A. Nielsen, I. Dybkjaer, Ammonia - Catalysis and Manufacture, Springer Berlin 1995, Kapitel 6, Seiten 202-326; M. Appl, Ammonia. Principles and Industrial Practice, WILEY-VCH Verlag GmbH 1999.

Neben der Standardroute für die Bereitstellung des Synthesegases über die Dampfreformierung kann alternativ oder zusätzlich zu dem Synthesegas über die Dampfreformierung (als Beimischung) ein Gas ausgewählt aus einem Koksofengas, einem Hochofengas oder einem Abgas von Zementwerken, wobei das Gas gegebenenfalls einer Aufbereitung unterworfen wurde, als Synthesegas verwendet werden. Je nach Herkunft des Gases kann vor der Gaswäsche eine Aufbereitung des Gases gegebenenfalls zweckmäßig sein. Die optionale Aufbereitung kann z.B. die Abtrennung eines oder mehrerer störender Bestandteile aus dem Gas und/oder die Erhöhung des COz-Gehalts durch eine Wassergas-Shift-Reaktion beinhalten. Das gegebenenfalls aufbereitete Gas, ausgewählt aus einem Koksofengas, einem Hochofengas oder einem Abgas von Zementwerken, eignet sich als COz-Quelle und nach Aufbereitung und Abtrennung von COz, CO und weiteren sauerstoffhaltigen Komponenten gegebenenfalls auch für die Ammoniaksynthese.

Bei der üblichen Ammoniak-Synthese wird dieses Synthesegas einer Gaswäsche mit einer Waschflüssigkeit unterworfen, um COz zu entfernen. Der von COz weitgehend befreite Gasstrom wird einer Methanisierung unterworfen, in der restliches CO und COz in dem Synthesegas, die Katalysatorgifte für die Ammoniaksynthese darstellen, in Methan überführt werden. Das die Methanisierung verlassende Gas enthält im wesentlichen Wasserstoff und Stickstoff in einem Verhältnis von 3:1 und etwas Methan und kann nach Entfernung von noch darin enthaltenem Wasser für die eigentliche Ammoniaksynthese eingesetzt werden.

Wie bereits vorstehend ausgeführt ist bei dem erfindungsgemäßen Verfahren im Hinblick auf die Ammoniaksynthese erst bei der COz-Wäsche gegebenenfalls eine Änderung der Prozessführung erforderlich. Im Folgenden werden zweckmäßige Ausführungsformen für die Entfernung von COz aus dem Synthesegas zum Erhalt einer mit COz beladenen Flüssigkeit für das erfindungsgemäße Verfahren beschrieben.

### Mit CO₂ beladene Methanolphase

In einer besonders bevorzugten Ausführungsform wird als Waschflüssigkeit für die Entfernung bzw. Absorption von COz aus dem Synthesegas eine Methanolphase als Waschflüssigkeit verwendet. Die Methanolphase ist bevorzugt Methanol. Die Methanolphase kann aber gegebenenfalls neben Methanol einen geringen Anteil an Verunreinigungen, wie Wasser oder organische Lösungsmittel, enthalten, aber bevorzugt in einer Menge von weniger als 10 Vol.%, bevorzugt weniger als 1 Vol.-%. Verunreinigungen können bei den anschließenden Reaktionen zu Nebenprodukten oder zu verringerten Umsätzen führen, weswegen sie möglichst weitgehend vermieden werden sollten.

Ein besonders geeignetes Verfahren ist der sogenannte Rectisol-Prozess von den Firmen Lurgi und Linde, bei dem Methanol als Waschflüssigkeit für die Gaswäsche verwendet wird. Um eine gute Absorption von COz in Methanol zu erreichen, wird insbesondere kaltes Methanol verwendet. Das Methanol bzw. die Methanolphase wird z.B. vor der Gaswäsche auf Temperaturen unter 0 °C, bevorzugt unter -20 °C, z.B. im Bereich von -20 °C bis -50 °C, bevorzugt -30 °C bis -40 °C abgekühlt. Hierfür kann eine NH₃-Kompressionskältemaschine genutzt werden, die Temperaturen von etwa -33 °C erreicht, was eine wirtschaftliche Methode darstellt. Es ist aber durchaus möglich, mit geeigneten Anlagen das Methanol auf noch tiefere Temperaturen abzukühlen, wodurch bessere Ergebnisse erzielt werden können, was aber mit der Wirtschaftlichkeit abzuwägen ist. Während der Gaswäsche erwärmt sich das Methanol wieder, z.B. auf etwa -20 °C, bleibt aber in der Regel unter 0°C und muss nach dem Rezyklieren wieder gekühlt werden. Die Gaswäsche des Synthesegases zur Entfernung von COz aus dem Synthesegas in die Waschflüssigkeit (Methanol bzw. Methanolphase) kann z.B. bei einem Druck im Bereich von 20 bis 50 bar durchgeführt werden.

Die nach der Gaswäsche erhaltene Waschflüssigkeit ist ein mit physikalisch absorbiertem COz beladene Methanolphase, die, ohne diese zu regenerieren in dem Umfang zu der nachstehend beschriebenen Formamid-Synthese geleitet werden kann.

In einer alternativen Ausführungsform kann eine andere Waschflüssigkeit für die Entfernung bzw. Absorption von COz aus dem Synthesegas verwendet werden, wobei in diesem Fall nach der Gaswäsche das COz durch Desorption aus der beladenen Waschflüssigkeit zu entfernen und durch Absorption in eine Methanolphase zu überführen ist. Es können alle Waschflüssigkeiten verwendet werden, die im Stand der Technik für die Gaswäsche zur Entfernung von COz aus einem Synthesegas bekannt sind, abgesehen von einer Methanolphase und einer wässrigen Ammoniaklösung, die sinnvollerweise wie beschrieben direkt einsetzbar sind.

Beispiele für übliche Waschflüssigkeiten sind Propylencarbonat (Fluor Solvent-Prozess der Firma Fluor Daniel), eine Mischung aus Dimethylethern und Polyethylenglykol (Selexol^{®} von der Firma Union Carbide), Alkanolamine, meist als wässrige Lösung, wie z.B. Monoethanolamin (MEA), Diglycolamin (DGA), Triethanolamin (TEA) und Methyldiethanolamin (MDEA), wässrige Ammoniaklösung und wässrige Kaliumcarbonat-Lösung.

Für diese alternative Ausführungsform wird als Waschflüssigkeit bevorzugt wässriges MDEA eingesetzt, dem mindestens ein Aktivator, wie z.B. MEA oder Diethanolamin (DEA), N-Methylaminoethanol (Monomethyl-MEA) oder Piperazin, zugesetzt ist. Bevorzugt ist dabei sogenanntes aktiviertes MDEA (aMDEA, dieser Prozess wird jetzt als OASE white bezeichnet), welches wässriges MDEA mit einem Zusatz von Monomethyl-MEA oder Piperazin darstellt und von der Firma BASF vertrieben wird. Eine andere bevorzugte Waschflüssigkeit ist wässrige Kaliumcarbonat-Lösung (Benfield-Wäsche von UOP).

Die nach der Gaswäsche erhaltene Waschflüssigkeit auf Basis der Waschflüssigkeiten nach dieser alternativen Ausführungsform ist eine mit chemisch und/oder physikalisch gebundenem Kohlendioxid beladene Waschflüssigkeit, die keine Methanolphase ist. Das darin enthaltene COz wird daher aus der beladenen Waschflüssigkeit desorbiert und das freigesetzte COz, vorzugsweise in dem für die nachstehend beschriebene Formamid-Synthese benötigten Umfang, in eine Methanolphase absorbiert, um eine mit COz beladene Methanolphase zu erhalten.

Die Desorption von COz kann durch die Standardmethoden erfolgen, die z.B. auf einer Erhöhung der Temperatur, einer Verringerung des Drucks und/oder eines verringerten Anteils von COz in der Gasphase über der Lösung beruhen. Konkret kann dies durch Auskochen der Lösung, Druckabsenkung durch Entspannen oder den Einsatz einer Vakuumpumpe und das Hindurchleiten eines Strippgases oder -dampfes erfolgen.

Die nach der beschriebenen Desorptions- und Absorptionsvorgängen erhaltene Flüssigkeit ist eine mit physikalisch absorbiertem COz beladene Methanolphase, die grundsätzlich der vorstehend beschriebenen, mit physikalisch absorbiertem COz beladenen und direkt einsetzbaren Methanolphase als Waschflüssigkeit im Wesentlichen entspricht, so dass die weiteren Verfahrensstufen gleich sind.

Die mit COz beladene Methanolphase gemäß den beiden obigen Ausführungsformen wird anschließend gemäß Variante a1) von Schritt a) als COz enthaltender Strom mit einem Wasserstoff enthaltenden Strom in Anwesenheit eines Katalysators unter Bildung von Methylformiat umgesetzt (vgl. nachstehende Reaktionsgleichung (Gl.4)) und das gebildete Methylformiat wird mit einem Ammoniak enthaltenden Strom unter Bildung von Formamid umgesetzt (vgl. nachstehende Reaktionsgleichung (Gl.5)). Das gebildete Formamid wird anschließend gemäß Schritt b) mit Ammoniak in Anwesenheit eines Katalysators umgesetzt, um Harnstoff zu erhalten (vgl. nachstehende Reaktionsgleichung (Gl. 6)). Bei den in der Anmeldung angeführten Reaktionsgleichungen ist die Stöchiometrie nicht berücksichtigt.

Die nachfolgenden Reaktionsgleichungen (4) bis (7) geben die Synthese wieder, wobei Kat. Katalysator bedeutet.

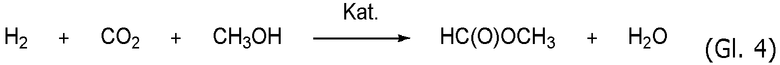

HC(O)OCH₃ + NH₃ → HC(O)NH₂ + CH₃OH (Gl. 5)

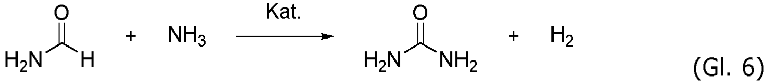

### Umsetzung von CO₂ und H₂ in der Methanolphase unter Bildung von Methylformiat gemäß Gl. 4

Die Reaktion gemäß Gl. 4 läuft in einer Methanolphase bzw. einer Methanollösung ab. Methanol agiert dabei als Lösungsmittel und als Reaktant gleichzeitig. Bei der Umsetzung von Kohlendioxid aus dem COz enthaltenden Strom und Wasserstoff aus dem Wasserstoff enthaltenden Strom in der Methanolphase in Anwesenheit eines Katalysators wird Methylformiat gebildet, das auch als Ameisensäuremethylester bezeichnet wird.

Die katalytische Umsetzung gemäß Gl. 4 und dafür geeignete Katalysatoren sind dem Fachmann bekannt. Es können literaturbekannte Katalysatoren für diese Reaktion verwendet werden, z.B. Gold, insbesondere ungeträgertes Gold oder Gold auf einem Träger, wie z.B. TiO₂ oder Al₂O₃, wie z.B. in der WO 2013/014160 A1 beschrieben, Kupfer-Katalysatoren, Cobalt-Katalysatoren oder Iridium-Katalysatoren, z.B. Ir-Katalysatoren, die einen mindestens zweizähnigen Phosphinliganden enthalten, wie z.B. in DE 102012019441 A1 beschrieben, oder der von Vaska et al., in DTIC Document AD-A199 861, 1988 beschriebene Katalysator [Ir(Cl)(CO)(Ph₃P)₂]. Geeignete Katalysatoren werden auch in der US 2012/071690 A1 beschrieben.

Ruthenium-Katalysatoren, wie z.B. Ruthenium-Phosphin-Komplexe, sind z.B. als Katalysatoren für die Umsetzung gemäß Gl. 4 geeignet. Daher kann z.B. ein Ruthenium-Katalysator, wie z.B. ein Ruthenium-Phosphin-Komplex, als Katalysator für die Umsetzung der mit COz beladenen Methanolphase und dem Wasserstoff enthaltenden Strom zur Bildung von Methylformiat gemäß Variante a1) verwendet werden. Dimethoxymethan kann sich als Nebenprodukt bilden, aber gewöhnlich in geringen Mengen in Abhängigkeit von den Reaktionsbedingungen.

Beispiele für Ruthenium-Phosphin-Komplexe als Katalysator für diese Umsetzung werden weiter unten beschrieben.

Der Katalysator, insbesondere der Ruthenium-Phosphin-Komplex, kann bei der katalytischen Umsetzung von Kohlendioxid und Wasserstoff in der Methanolphase zu Methylformiat als homogener Katalysator oder als immobilisierter Katalysator eingesetzt werden. Die katalytische Umsetzung mit dem Katalysator, z.B. dem Ruthenium-Phosphin-Komplex, kann homogen oder heterogen durchgeführt werden, z.B. mit einem immobilisierten Katalysator in einem Festbettreaktor oder mit einem gelösten Katalysator in einem Flüssigbettreaktor.

Die katalytische Umsetzung von Kohlendioxid und Wasserstoff in der Methanolphase zu Methylformiat kann kontinuierlich oder chargenweise durchgeführt werden, wobei ein kontinuierlicher Betrieb bevorzugt ist. Die katalytische Umsetzung wird vorzugsweise in einem Autoklav oder einem Druckreaktor durchgeführt. Ein Autoklav eignet sich für den Chargenbetrieb. Ein Druckreaktor eignet sich für den kontinuierlichen Betrieb.

Geeignete Reaktionsbedingungen für die für diese Reaktion bekannten Katalysatoren sind dem Fachmann bekannt. Im Folgenden werden insbesondere Einzelheiten für geeignete Reaktionsbedingungen beim Einsatz von Ruthenium-Phosphin-Komplexen als Katalysator angeführt. Sofern die folgenden Absätze nicht ausdrücklich auf den Ruthenium-Phosphin-Komplex Bezug nehmen, gelten sie nicht nur für den Ruthenium-Phosphin-Komplex, sondern auch bei Einsatz anderer geeigneter Katalysatoren.

Die Konzentration an Ruthenium-Phosphin-Komplex als Katalysator bei der katalytischen Umsetzung von Kohlendioxid und Wasserstoff in der Methanolphase zu Methylformiat kann z.B. im Bereich von 0,1 mmol-% bis 5,0 mol-%, bevorzugt von 1,0 mmol-% bis 1,0 mol-%, besonders bevorzugt 2,0 mmol-% bis 0,1 mol-%, bezogen auf die molare Menge an COz, liegen.

Die katalytische Umsetzung von Kohlendioxid und Wasserstoff in der Methanolphase zu Methylformiat, insbesondere bei Einsatz des Ruthenium-Phosphin-Komplexes als Katalysator, erfolgt ferner bevorzugt in Anwesenheit einer Säure. Die Säure dient dabei als Co-Katalysator zur Aktivierung des Katalysators und des Substrates und kann die Ausbeute der Reaktion verbessern.

Die Säure kann eine protische Säure (Brønsted-Säure) oder eine Lewis-Säure sein, wobei eine Lewis-Säure bevorzugt ist. Die Säure kann eine organische Säure oder eine anorganische Säure sein.

Unter einer Lewis-Säure versteht man einen Elektronenpaarakzeptor, d.h. ein Molekül oder Ion mit unvollständiger Edelgaskonfiguration, das ein von einem anderen Molekül (Lewis-Base) zur Verfügung gestelltes Elektronenpaar aufnehmen und mit dieser ein sogenanntes Lewis-Addukt bilden kann.

Beispiele für zweckmäßige Säuren, wie Lewis-Säuren und protische Säuren, sind Organoaluminium-Verbindungen, wie z.B. Aluminiumtriflat, (Aluminumtris(trifluoromethansulfonat), Al(OTf)₃ (Tf = -SO₂CF₃)) und Aluminiumtriacetat, Organobor-Verbindungen, wie z.B. Tris(pentafluorophenyl)boran, Bi(OTf)₃, 2,4,6-Trimethylbenzoesäure, Sulfonsäuren, wie z.B. p-Toluolsulfonsäure, Bis(trifluormethan)sulfonimid (HNTf₂), Scandiumverbindungen, wie z.B. Scandiumtriflat, perfluorierte Copolymere, die mindestens eine Sulfogruppe aufweisen, wie sie z.B. unter dem Handelsnamen Nafion^{®} NR50 erhältlich sind, oder Kombinationen davon.

Das Molverhältnis von Ruthenium-Phosphin-Komplex zu Säure kann z.B. im Bereich von 1:800 bis 1:1, bevorzugt von 1:80 bis 1:5, besonders bevorzugt von 1:50 bis 1:10 liegen.

Die katalytische Umsetzung von Kohlendioxid und Wasserstoff in Anwesenheit des Katalysators findet in einer Methanolphase statt. Das Methanol dient dabei gleichzeitig als Reaktant und als Lösungsmittel. Die Methanolphase ist bevorzugt Methanol. Die Methanolphase kann aber gegebenenfalls neben Methanol einen geringen Anteil an Verunreinigungen, wie Wasser oder organische Lösungsmittel, enthalten, aber bevorzugt in einer Menge von weniger als 10 Vol.%, bevorzugt weniger als 1 Vol.-%.

Der Ruthenium-Phosphin-Komplex liegt in der Methanolphase bevorzugt zumindest teilweise in Lösung vor. Die katalytische Umsetzung von Kohlendioxid und Wasserstoff in Methanol ist vorzugsweise eine homogene katalytische Reaktion. Die homogene Katalyse kann mildere Reaktionsbedingungen und höhere Selektivitäten ermöglichen.

Methanol wird in sehr hohem Überschuss verwendet, da es auch als Lösungsmittel verwendet wird. Ein stöchiometrischer Überschuss an H₂ gegenüber COz ist bevorzugt. Beispielsweise kann für die katalytische Umsetzung das Verhältnis von p(CO₂) : p(H₂) im Bereich von 2:1 bis 1:10, bevorzugt im Bereich von 1:1 bis 1:5, bevorzugter 1:1,1 bis 1:3 zweckmäßig sein, wobei p der Partialdruck des jeweiligen Edukts bei 23 °C ist. Konkrete Beispiele für geeignete Verhältnisse von p(CO₂) : p(H₂) sind z.B. 1:2 oder 2:3.

Die katalytische Umsetzung von Kohlendioxid und Wasserstoff in der Methanolphase, insbesondere bei Einsatz des Ruthenium-Phosphin-Komplex als Katalysator, erfolgt bevorzugt bei einer Temperatur im Bereich von Raumtemperatur (z.B. 20°C) bis 150 °C und besonders bevorzugt im Bereich von 60 bis 140 °C oder von 80 bis 120 °C, wobei die besten Ergebnisse bei etwa 100 °C erzielt werden.

Die katalytische Umsetzung von Kohlendioxid und Wasserstoff in der Methanolphase, insbesondere bei Einsatz des Ruthenium-Phosphin-Komplex als Katalysator, erfolgt bevorzugt bei einem Druck im Bereich von 40 bar bis 220 bar, bevorzugter im Bereich von 80 bar bis 200 bar, wobei die besten Ergebnisse bei etwa 100 bis 180 bar erzielt werden.

Die geeignete Reaktionsdauer für die katalytische Umsetzung von Kohlendioxid und Wasserstoff in der Methanolphase kann in Abhängigkeit von den anderen Reaktionsparametern variieren. Zweckmäßig liegt die Reaktionsdauer der Umsetzung z.B. in einem Bereich von 3 bis 20 Stunden, bevorzugt 12 bis 18 Stunden.

Bei der Umsetzung von Kohlendioxid und Wasserstoff in einer Methanolphase in Anwesenheit eines Katalysators, wie eines Ruthenium-Phosphin-Komplexes als Katalysator, und gegebenenfalls einer Säure unter Bildung von Methylformiat und Wasser wird insbesondere ein Methylformiat enthaltendes Reaktionsgemisch erhalten, welches gewöhnlich nach Ablassen von nicht umgesetztem H₂ und COz für die Ammonolyse zur Bildung des Formamids gemäß Gl. 5 verwendet wird.

Zum Ablassen von nicht umgesetztem H₂ und COz werden zunächst insbesondere der Druck und gegebenenfalls die Temperatur gesenkt, um die gasförmigen Komponenten (H₂ und COz) möglichst weitgehend von dem Methylformiat enthaltenden Reaktionsgemisch zu entfernen, bevor Ammoniak zugesetzt wird. Bei welchem Druck die gasförmigen Komponenten abgetrennt werden, hängt von den vorliegenden Drücken der gekoppelten Prozessstufen ab.

Je nach eingesetztem Verhältnis von H₂ zu COz kann sich gegebenenfalls eine stufenweise Druckabsenkung bzw. Entspannung lohnen. Bei der ersten Entspannung wird hauptsächlich H₂ bzw. H_{2/}N₂ abgetrennt, etwas COz wird dabei ebenfalls entfernt. Dieser Stoffstrom kann rezykliert werden. Die letzte Entspannungsstufe, die gegebenenfalls durch Strippen mit Strippgas unterstützt wird, eignet sich eher zur Verbrennung im Primärreformer bzw. Entsorgung. Der Vorteil einer stufenweisen Entspannung besteht darin, dass die abgetrennten Gase noch unter Druck stehen und sich so besser für die Rezyklierung eignen.

Das Methylformiat enthaltende Reaktionsgemisch, von dem nicht umgesetztes H₂ und COz abgetrennt wurde, kann z.B. über folgende zwei Verfahrensführungen für die Ammonolyse gemäß Gl. 5 eingesetzt werden. Das gebildete Methylformiat kann aus dem Reaktionsgemisch abgetrennt werden und in einer separaten Reaktion mit Ammoniak umgesetzt werden. Die Abtrennung kann z.B. einfach durch Destillation erfolgen, wobei das Methylformiat die Komponente mit dem niedrigsten Siedepunkt ist. In einer alternativen Ausführungsform kann das gebildete Methylformiat ohne vorherige Abtrennung aus dem Reaktionsgemisch direkt mit Ammoniak umgesetzt werden. In diesem Fall wird der Ammoniak einfach nach erfolgter katalytischer Umsetzung und nach Ablassen der nicht umgesetzten Reaktanten CO₂ und H₂ in das das gebildete Methylformiat enthaltende Reaktionsgemisch eingeleitet.

Das bei der katalytischen Umsetzung von Kohlendioxid und Wasserstoff in Methanol gebildete Methylformiat wird anschließend einer Ammonolyse mit Ammoniak zur Bildung des Formamids unterworfen. Die folgenden Ausführungen zur Umsetzung des Methylformiats mit Ammoniak gelten unabhängig davon, ob hierfür das Methylformiat enthaltende Reaktionsgemisch oder das aus dem Reaktionsgemisch abgetrennte Methylformiat wie vorstehend beschrieben verwendet wird.

### Umsetzung von Methylformiat und NH₃ unter Bildung von Formamid gemäß Gl. 5

Nach der Bildung von Methylformiat gemäß Gl. 4 findet eine Ammonolyse des selbigen durch Umsetzung mit Ammoniak unter Bildung von Formamid statt, wie in Gl. 5 dargestellt. Dabei findet eine nukleophile Substitution statt und es wird Methanol freigesetzt. Das freigesetzte Methanol wird rezykliert, so dass Formamid damit das überwiegende Reaktionsprodukt darstellt. Wie angeführt, kann Dimethoxymethan als Nebenprodukt gebildet werden, aber gewöhnlich in geringen Mengen.

Bei der Reaktion gemäß Gl. 5 handelt es sich um eine allgemein bekannte Reaktion. Die Reaktionsbedingungen sind dem Fachmann gut bekannt. Die Umsetzung des Methylformiats mit Ammoniak zum Formamid läuft in der Regel quantitativ ab und ist dem Fachmann gut bekannt. Bei der herkömmlichen Formamidsynthese ausgehend von CO und MeOH wird sie ebenfalls ausgeführt. Die Umsetzung des Methylformiats mit Ammoniak zum Formamid erfordert keinen Katalysator, wobei der Einsatz eines Katalysators aber nicht ausgeschlossen wird.

Die Umsetzung des Methylformiats mit Ammoniak zu Formamid und Methanol kann z.B. bei einer Temperatur im Bereich von Raumtemperatur (z.B. 20°C) bis 100 °C, besonders bevorzugt bei 60 bis 80 °C, durchgeführt werden. Die Umsetzung kann z.B. bei einem Druck im Bereich von 1 bar (bzw. Atmosphärendruck) bis 70 bar, bevorzugt im Bereich von 1 bar (bzw. Atmosphärendruck) bis 45 bar durchgeführt werden.

Nach der Umsetzung werden das Methanol und gegebenenfalls Edukte abdestilliert. Formamid bleibt als Rückstand zurück.

Das Methanol, das bei der Umsetzung von Methylformiat mit dem Ammoniak enthaltenden Strom zur Bildung von Formamid gebildet wird, kann bevorzugt für die Waschflüssigkeit oder die Methanol enthaltende Flüssigkeit in dem Verfahren wiederverwendet werden.

### Umsetzung von Formamid und Ammoniak unter Bildung von Harnstoff gemäß Gl. 6 oder Umsetzung von Formamid unter Bildung von Harnstoff

Die folgende Umsetzung des gebildeten Formamids mit Ammoniak gemäß Gl. 6 in Anwesenheit eines Katalysators führt schließlich zur Bildung von Harnstoff und Wasserstoff. Die Wiederverwendbarkeit des Wasserstoffes ist ein besonderer Vorteil des erfindungsgemäßen Verfahrens. Der bei der Reaktion freigesetzte Wasserstoff fällt bei dem für die Reaktion eingesetzten Druck an. Er kann in die Formamid-Synthese oder in die Ammoniak-Synthese zurückgeführt werden.

In einer alternativen Ausführungsform kann die Umsetzung des gebildeten Formamids in Anwesenheit eines Katalysators unter Bildung von Harnstoff und Wasserstoff auch ohne Zugabe von Ammoniak durchgeführt werden. Auch in diesem Fall wird wiederverwendbarer Wasserstoff gebildet.

Dementsprechend umfasst die katalytische Synthese von Harnstoff bevorzugt die Umsetzung von Formamid mit Ammoniak in Anwesenheit eines Katalysators, z.B. eines Ruthenium-Phosphin-Komplexes, unter Bildung von Harnstoff und Wasserstoff. Alternativ umfasst die katalytische Synthese von Harnstoff die Umsetzung von Formamid in Anwesenheit eines Katalysators, z.B. eines Ruthenium-Phosphin-Komplexes, unter Bildung von Harnstoff und Wasserstoff, wobei bei dieser Alternative auch CO gebildet wird. Bei der alternativen Variante wird nur Formamid als Ausgangsmaterial für die katalytische Synthese bzw. Umsetzung in Anwesenheit eines Katalysators zur Bildung von Harnstoff eingesetzt, insbesondere wird kein NH₃ zur Reaktionsmischung zugegeben. Als Ausgangsmaterialien für die Synthese werden daher Formamid oder bevorzugt Formamid und Ammoniak eingesetzt.

Sofern nicht anders angegeben, beziehen sich die Erläuterungen zur katalytischen Synthese sowohl auf die bevorzugte Variante als auch auf die alternative Variante, die vorstehend angeführt sind. Es versteht sich, dass Angaben, die den zugegebenen Ammoniak betreffen, sich nur auf die bevorzugte Variante beziehen.

Soll Wasserstoff zurück in die Formamid-Synthese geführt werden, so muss der Druck der Harnstoff-Synthese leicht oberhalb des Drucks für die Formamidsynthese liegen, in diesem Fall für die Reaktion gemäß Gl. 4 (siehe vorstehend). Dann wird das Produktionspaar Formamid-Harnstoff im stationären Zustand zum Selbstversorger: Wasserstoff, der aus der Harnstoffsynthese gemäß Gl. 6 oder aus der alternativen Synthese ohne Zugabe von Ammoniak freigesetzt wird, wird für die Synthese von Formamid gemäß Gl. 4/5 verwendet. Lediglich beim Start-up oder zum Ausgleich von H₂-Verlusten wird Ammoniak-Synthesegas (N₂/H₂) benötigt.

Wird der Wasserstoff in die Ammoniak-Synthese zurückgeführt, so kann der Wasserstoff direkt in den High-Pressure-Loop der Synthese oder an beliebige Saugstufen des Syngaskompressors mit oder ohne N₂ eingeleitet werden. Falls nötig wird dementsprechend der Druck der Harnstoff-Synthese angepasst. Die Saugstufen des Kompressors (Syngas) können z.B. Drücke von 32, 66, 109 und 195 bar aufweisen.

Als Katalysator für die Umsetzung des gebildeten Formamids mit Ammoniak zur Bildung von Harnstoff gemäß Gl. 6 oder für die Umsetzung des gebildeten Formamids zur Bildung von Harnstoff können geeignete Katalysatoren verwendet werden. In einer bevorzugten Ausführungsform wird ein Ruthenium-Katalysator, insbesondere ein Ruthenium-Phosphin-Komplex, als Katalysator für die Umsetzung des gebildeten Formamids oder des gebildeten Formamids mit Ammoniak zur Bildung von Harnstoff verwendet.

Beispiele für besonders geeignete Ruthenium-Phosphin-Komplexe als Katalysator für diese Umsetzung werden weiter unten beschrieben.

Im Folgenden werden insbesondere Einzelheiten für geeignete Reaktionsbedingungen beim Einsatz von Ruthenium-Phosphin-Komplexen als Katalysator angeführt. Sofern die folgenden Absätze nicht ausdrücklich auf den Ruthenium-Phosphin-Komplex Bezug nehmen, gelten sie nicht nur für den Ruthenium-Phosphin-Komplex, sondern auch bei Einsatz anderer geeigneter Katalysatoren.

Der Katalysator, insbesondere ein Ruthenium-Phosphin-Komplex, kann bei der katalytischen Umsetzung von Formamid oder von Formamid und Ammoniak zu Harnstoff als homogener Katalysator oder als immobilisierter Katalysator eingesetzt werden. Auch Zweiphasen-Systeme mit Phasentransferkatalyse sind möglich. Die katalytische Umsetzung mit dem Katalysator, insbesondere dem Ruthenium-Phosphin-Komplex, kann homogen oder heterogen durchgeführt werden, z.B. mit einem immobilisierten Katalysator in einem Festbettreaktor oder mit einem gelösten Katalysator in einem Flüssigbettreaktor.

Die katalytische Umsetzung von Formamid oder von Formamid und Ammoniak kann kontinuierlich oder chargenweise durchgeführt werden, wobei ein kontinuierlicher Betrieb bevorzugt ist. Die katalytische Umsetzung wird vorzugsweise in einem Autoklav oder einem Druckreaktor durchgeführt. Ein Autoklav eignet sich für den Chargenbetrieb. Ein Druckreaktor eignet sich für den kontinuierlichen Betrieb.

Die katalytische Umsetzung von Formamid oder bevorzugt von Formamid mit Ammoniak kann gegebenenfalls ferner in Anwesenheit einer Säure als Co-Katalysator durchgeführt werden, wobei es sich um eine Brønsted-Säure oder eine Lewis-Säure handeln kann. Die Säure kann eine organische Säure oder eine anorganische Säure sein. Die Säure kann dabei zur zusätzlichen Aktivierung des Katalysators bzw. des Formamids führen und die Ausbeute der Reaktion verbessern.

Beispiele für zweckmäßige Brønsted-Säuren oder Lewis-Säuren sind Organoaluminium-Verbindungen, wie z.B. Aluminiumtriflat (Aluminumtris(trifluoromethansulfonat)) und Aluminiumtriacetat, Organobor-Verbindungen, wie z.B. Tris(pentafluorophenyl)boran, Sulfonsäuren, wie z.B. p-Toluolsulfonsäure, Bis(trifluormethan)sulfonimid (HNTf₂), Scandiumverbindungen, wie z.B. Scandiumtriflat, perfluorierte Copolymere, die mindestens eine Sulfogruppe aufweisen, wie sie z.B. unter dem Handelsnamen Nafion^{®} NR50 erhältlich sind, oder Kombinationen davon.

Die katalytische Umsetzung von Formamid und Ammoniak zu Harnstoff oder die katalytische Umsetzung von Formamid zu Harnstoff erfolgt z.B. bei einer Temperatur im Bereich von 50 bis 250 °C, bevorzugt im Bereich von 120 bis 200 °C, besonders bevorzugt im Bereich von 140 bis 170 °C.

Die katalytische Umsetzung von Formamid oder von Formamid mit Ammoniak zu Harnstoff erfolgt z.B. bei einem Druck (Reaktionsdruck) im Bereich von Umgebungsdruck bis 150 bar, bevorzugt im Bereich von 2 bar bis 60 bar, besonders bevorzugt im Bereich von 5 bis 40 bar. Die Umsetzung kann bei der bevorzugten Variante gegebenenfalls unter Bedingungen erfolgen, bei denen flüssiges oder überkritisches Ammoniak vorliegt (kritischer Druck (NH₃) = 113 bar; kritische Temperatur (NH₃) = 132,5 °C), das als Lösungsmittel fungieren kann.

In der bevorzugten Variante kann die bei der Umsetzung eingesetzte Menge an Ammoniak in Äquivalenten (äq.) bezogen auf Formamid kann z.B. im Bereich von 1 bis 300 äq., bevorzugt von 4 äq. bis 100 äq., besonders bevorzugt von 29 bis 59 äq. liegen.

In einer bevorzugten Ausführungsform erfolgt die Umsetzung mit etwa 29 bis 59 äq. Ammoniak bezogen auf Formamid bei einem Druck im Bereich von 5 bis 40 bar, insbesondere 10 bis 30 bar. Besonders bevorzugt eingesetzte Lösungsmittel sind dabei Dioxan, insbesondere 1,4-Dioxan, oder Toluol.

Die Umsetzung erfolgt demnach vorzugsweise mit einem hohen stöchiometrischen Überschuss an Ammoniak. Dadurch kann die Ausbeute an Harnstoff verbessert werden.

Die geeignete Reaktionsdauer für die katalytische Umsetzung von Formamid oder von Formamid mit Ammoniak kann in Abhängigkeit von den anderen Reaktionsparametern variieren. Zweckmäßig liegt die Reaktionsdauer der Umsetzung z.B. in einem Bereich von 1 Minute bis 24 Stunden oder 30 Minuten bis 24 Stunden, bevorzugt 3 bis 15 Stunden, besonders bevorzugt 6 bis 10 Stunden.

Bei dem erfindungsgemäßen Verfahren kann die Umsetzung von Formamid oder bevorzugt von Formamid mit Ammoniak in Abwesenheit oder Anwesenheit von Lösungsmittel, insbesondere organischem Lösungsmittel, durchgeführt werden. Bei Abwesenheit von Lösungsmittel kann gegebenenfalls im Überschuss vorliegendes Ammoniak in Form von flüssigem oder bevorzugt überkritischem Ammoniak als Lösungsmittel fungieren.

In einer bevorzugten Ausführungsform wird die Umsetzung in einem Lösungsmittel, insbesondere einem organischen Lösungsmittel, durchgeführt. Es kann ein Lösungsmittel oder ein Gemisch von zwei oder mehr Lösungsmitteln eingesetzt werden, wobei vorzugsweise ein Lösungsmittel eingesetzt wird.

Das Lösungsmittel ist vorzugsweise ein organisches Lösungsmittel, insbesondere ein aprotisches organisches Lösungsmittel. Das Lösungsmittel kann polar oder unpolar sein, wobei unpolare organische Lösungsmittel bevorzugt sind. Das Lösungsmittel wird bevorzugt so gewählt, dass der eingesetzte Katalysator, vorzugsweise der Ruthenium-Phosphin-Komplex, zumindest teilweise darin gelöst werden kann.

Das Lösungsmittel wird bevorzugt ausgewählt aus der Gruppe bestehend aus cyclischen und nicht-cyclischen Ethern, substituierten und unsubstituierten Aromaten, Alkanen und halogenierten Kohlenwasserstoffen, wie z.B. Di- und Trichlormethan, wobei das Lösungsmittel bevorzugt aus halogenierten Kohlenwasserstoffen, cyclischen Ethern und substituierten oder unsubstituierten Aromaten, bevorzugt aus cyclischen Ethern und substituierten oder unsubstituierten Aromaten, ausgewählt wird. Beispiele für Aromaten sind Benzol oder Benzol, das einen oder mehrere aromatische Substituenten (z.B. Phenyl) und/oder aliphatische Substituenten (z.B. C₁-C₄-Alkyl) aufweist. Besonders bevorzugte Lösungsmittel sind Dioxan, insbesondere 1,4-Dioxan, Toluol, und Tetrahydrofuran (THF). Aber auch Dichlormethan oder Trichlormethan können mit Vorteil eingesetzt werden.

Als Lösungsmittel können alternativ gegebenenfalls auch ionische Flüssigkeiten eingesetzt werden. Ionische Flüssigkeiten sind dem Fachmann bekannt. Es handelt sich um Salze, die bei niedrigen Temperaturen, z.B. bei Temperaturen von nicht mehr als 100 °C, flüssig sind. Das Kation der ionischen Flüssigkeit ist z.B. ausgewählt aus Imidazolium, Pyridinium, Pyrrolidinium, Guanidinium, Uronium, Thiouronium, Piperidinium, Morpholinium, Ammonium und Phosphonium, wobei dieses Kation bevorzugt mit einer oder mehreren Alkylgruppen substituiert sein kann. Das Anion der ionischen Flüssigkeit ist z.B. ausgewählt aus Halogeniden, Tetrafluoroboraten, Trifluoracetaten, Triflaten, Hexafluorophosphaten, Phosphinaten, Tosylaten oder organische Ionen, wie z.B. Imiden oder Amiden.

Der Katalysator, insbesondere der Ruthenium-Phosphin-Komplex, liegt in dem Lösungsmittel bevorzugt zumindest teilweise oder vollständig in Lösung vor. Die katalytische Umsetzung von Formamid oder von Formamid mit Ammoniak zu Harnstoff ist vorzugsweise eine homogene katalytische Reaktion. Dabei liegen Katalysator und Edukte in Lösung, also in derselben Phase vor. Die homogene Katalyse kann mildere Reaktionsbedingungen und gegebenenfalls höhere Selektivitäten und höhere Wechselzahlen (Umsatzzahl TON ("turnover number") und/oder Umsatzfrequenz TOF ("turnover frequency")) ermöglichen.

Für den Fall, dass ein Lösungsmittel oder ein Lösungsmittelgemisch verwendet wird, liegt die Konzentration des oder der Lösungsmittel z.B. in einem Bereich von 5 bis 500 mL, bevorzugt von 10 bis 300 mL, bevorzugter von 50 bis 250 mL, pro 1 mmol Ru-Phosphin-Komplex.

Die Konzentration an Katalysator, insbesondere an Ruthenium-Phosphin-Komplex als Katalysator, bei der Umsetzung kann z.B. im Bereich von 0,05 mol-% bis 10 mol-%, bevorzugt von 0,25 mol-% bis 5 mol-%, besonders bevorzugt 0,5 mol-% bis 2 mol-%, bezogen auf die molare Menge an Formamid, liegen.

Nach der Reaktion unter Bildung von Harnstoff werden die gasförmigen Komponenten entfernt. Dabei handelt es sich um freigesetzten Wasserstoff und gegebenenfalls überschüssiges Ammoniak. Der Druck soll dabei möglichst hoch bleiben, eine Druckabsenkung sollte daher vermieden werden. Die Temperatur kann dabei beliebig sein, bevorzugt sind aber hohe Temperaturen, damit mehr H₂/NH₃ ausgetrieben wird. Das gegebenenfalls vorhandene überschüssige NH₃ kann in einer Rückgewinnungsstufe vom Wasserstoff abgetrennt werden. Der Druck der Harnstoff-Synthese und damit der Druck des freigesetzten Wasserstoffs hängt davon ab, welche Verwendung für den freigesetzten Wasserstoff beabsichtigt wird, wie vorstehend beschrieben. Für die bessere Abtrennung der Gase kann gegebenenfalls Stickstoff, z.B. aus der Formamid-Synthese, als Strippmittel eingesetzt werden.

Das Gasgemisch aus der Harnstoffsynthese (H₂/NH₃) bei der Variante mit zugesetztem Ammoniak als Edukt wird auf ähnliche Art und Weise, wie es in der Ammoniak-Synthese üblich ist aufbereitet. Das abgetrennte NH₃ wird in der Harnstoff- und/oder Formamid-Synthese wiederverwendet und der abgetrennte Wasserstoff mit oder ohne Stickstoff kann je nachdem, ob Stickstoff als Strippmittel verwendet wurde, zum Synthesegas aufbereitet werden. Dass bei der Umsetzung von Formamid und Ammoniak zu Harnstoff Wasserstoff wieder freigesetzt wird und für die Ammoniak- oder Formamid-Synthese wiederverwendet werden kann, ist ein besonderer Vorteil des erfindungsgemäßen Verfahrens.

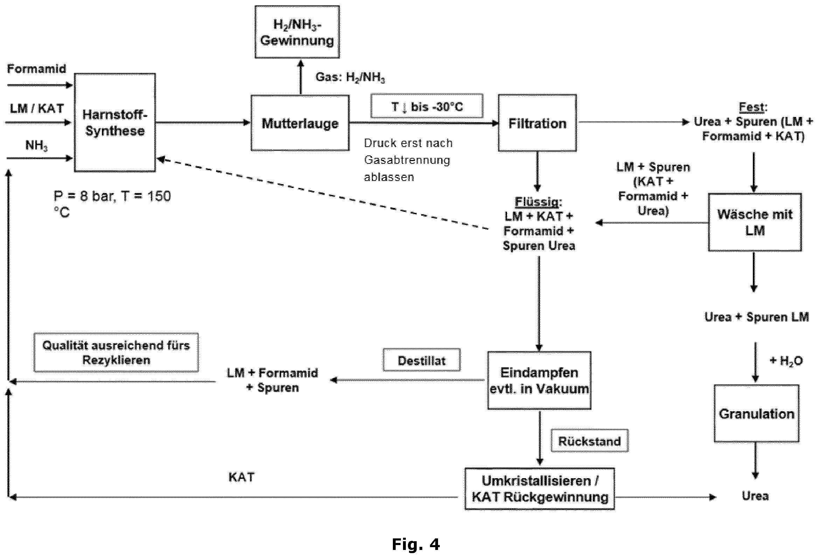

Der flüssige Reaktionsrückstand, enthaltend Harnstoff, Katalysator, gegebenenfalls überschüssiges Formamid und Spuren vom Ammoniak sowie gegebenenfalls Lösungsmittel, wird in die Aufbereitung geführt. Einzelne mögliche Aufbereitungsschritte werden nachstehend zu Fig. 4 näher beschrieben.

### Mit CO₂ beladene wässrige Ammoniaklösung

In einer weiteren alternativen Ausführungsform gemäß Variante a2) erfolgt die Synthese des Formamids nicht über Methylformiat, sondern über Ammoniumformiat als Zwischenprodukt.

Bei dieser alternativen Ausführungsform wird für die Gaswäsche des Synthesegases zur Entfernung von CO₂ als Waschflüssigkeit eine wässrige Ammoniaklösung, bevorzugt eine verdünnte wässrige Ammoniaklösung, verwendet, so dass CO₂ zumindest teilweise in Form eines entsprechenden Carbamat- und Carbonat-Gemisches in der Waschflüssigkeit gebunden wird (siehe nachfolgende Reaktionsgleichung Gl. 7). Hier ist darauf hinzuweisen, dass es sich um eine vereinfachte Reaktionsgleichung handelt, da COz und NH₃ miteinander und dem Wasser unter Bildung eines komplexen Gemisches von gelösten Salzen reagieren, das neben genannten Salzen weitere Verbindungen umfassen kann. Diese Reaktion findet in der CO₂-Wäsche statt, wobei hier hervorzuheben ist, dass in diesem Fall keine Regeneration des Waschmittels notwendig ist und die beladene Waschflüssigkeit direkt für die weitere Synthese verwendet werden kann.

CO₂ + H₂O + NH₃ → NH₄HCO₃ + (NH₄)₂CO₃ + H₂NCOONH₄ + H₂O (GI. 7)

Von den Reaktionsprodukten gemäß GI. 7 ist nur Ammoniumcarbamat für die weiteren Transformationen unerwünscht. Bei Temperaturerhöhung (ab etwa 50-60 °C) hydrolysiert aber Ammoniumcarbamat mit Wasser zu Ammoniumcarbonat. Daher ist es bevorzugt, dass die Ammoniaklösung möglichst verdünnt ist, da dann weniger Ammoniumcarbamat zu erwarten ist.

Die als Waschflüssigkeit eingesetzte wässrige Ammoniaklösung weist bevorzugt einen Ammoniak-Anteil im Bereich von 5 bis 60 Gew.-% auf. Bevorzugt wird dabei eine verdünnte wässrige Ammoniaklösung eingesetzt, deren Ammoniak-Anteil unter 30 Gew.-% beträgt. In einer bevorzugten Ausführungsform wird die Gaswäsche des Synthesegases zur Entfernung von CO₂ aus dem Synthesegas in die Waschflüssigkeit (wässrige Ammoniaklösung) bei einem Druck von 20 bis 50 bar und/oder bei einer Temperatur von unter 100 °C, bevorzugt im Bereich von 30 bis 70 °C, durchgeführt.

Diese mit chemisch und physikalisch gebundenem CO₂ beladene Waschflüssigkeit wird daher als CO₂ enthaltender Strom mit einem Wasserstoff enthaltenden Strom in Anwesenheit von einem Katalysator und gegebenenfalls organischem Lösungsmittel oder in Anwesenheit von einem Katalysator und einer Säure, z.B. einer Lewis-Säure, und gegebenenfalls organischem Lösungsmittel unter Bildung von Ammoniumformiat oder unter Bildung von Ammoniumformiat und Formamid umgesetzt (siehe nachstehende Reaktionsgleichung (Gl. 8). Dabei findet die katalytische Umsetzung von Ammoniak, Kohlendioxid und Wasserstoff zu Ammoniumformiat oder zu Formamid und Ammoniumformiat statt. Das gebildete Ammoniumformiat wird durch Wärmebehandlung in Formamid überführt (siehe nachstehende Reaktionsgleichung Gl. 9). Da die Reaktion insbesondere bei erhöhtem Druck und Temperatur durchgeführt wird, zersetzen sich die Salze, wie die Carbonate und Carbamate, in der Ammoniaklösung und setzen CO₂ frei. Die nachweisbare Zersetzung fängt schon bei etwa 60-70 °C an.

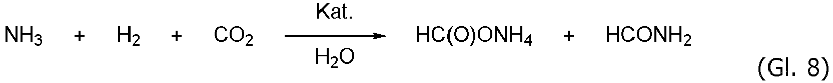

Je nach verwendeten Prozessparametern entstehen Ammoniumformiat und Formamid in verschiedenen Verhältnissen, wobei bei bestimmten Reaktionsbedingungen, z.B. einer niedrigen Temperatur, auch nur Ammoniumformiat gebildet werden kann. Unter thermischer Einwirkung (T) spaltet das Ammoniumformiat Wasser ab und bildet Formamid gemäß folgender Gl. 9. Damit ist Formamid im Prinzip das einzige Produkt der Reaktion.

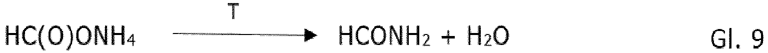

### Umsetzung von CO₂ und H₂ in wässriger Ammoniaklösung unter Bildung von Ammoniumformiat und gegebenenfalls Formamid gemäß Gl. 8

Die Reaktion gemäß Gl. 8 läuft in wässriger Ammoniaklösung ab. Die erhaltene Waschflüssigkeit, die mit COz und NH₃ zumindest teilweise in Form der Carbonate beladen ist, wird mit einem Wasserstoff-enthaltenden Strom in Anwesenheit eines Katalysators und gegebenenfalls von organischem Lösungsmittel oder in Anwesenheit eines Katalysators und einer Säure, insbesondere einer Lewis-Säure, und gegebenenfalls von organischem Lösungsmittel umgesetzt. Bei der Umsetzung wird Ammoniumformiat und gegebenenfalls Formamid gebildet.

Als Katalysator für die Umsetzung gemäß Gl. 8 können geeignete Katalysatoren und Katalysatortypen verwendet werden, die bereits vorher aufgeführt wurden. In einer bevorzugten Ausführungsform wird ein Ruthenium-Katalysator, insbesondere ein Ruthenium-Phosphin-Komplex, als Katalysator für die Umsetzung von NH₃, COz und H₂ zu Ammoniumformiat und gegebenenfalls Formamid verwendet. Insbesondere bei Einsatz eines Ruthenium-Phosphin-Komplexes als Katalysator wird bevorzugt eine Säure, insbesondere eine Lewis-Säure, als Co-Katalysator in Kombination verwendet.

Beispiele für besonders geeignete Ruthenium-Phosphin-Komplexe als Katalysator für diese Umsetzung werden weiter unten beschrieben.

Im Folgenden werden insbesondere Einzelheiten für geeignete Reaktionsbedingungen beim Einsatz von Ruthenium-Phosphin-Komplexen als Katalysator angeführt. Sofern die folgenden Absätze nicht ausdrücklich auf den Ruthenium-Phosphin-Komplex Bezug nehmen, gelten sie nicht nur für den Ruthenium-Phosphin-Komplex, sondern auch bei Einsatz anderer geeigneter Katalysatoren.

Der Katalysator, z.B. der Ruthenium-Phosphin-Komplex, kann bei der katalytischen Umsetzung von Ammoniak, Kohlendioxid und Wasserstoff zu Ammoniumformiat oder zu Formamid und Ammoniumformiat als homogener Katalysator oder als immobilisierter Katalysator eingesetzt werden. Auch Zweiphasen-Systeme mit Phasentransferkatalyse sind möglich. Die katalytische Umsetzung mit dem Katalysator, z.B. dem Ruthenium-Phosphin-Komplex, kann homogen oder heterogen durchgeführt werden, z.B. mit einem immobilisierten Katalysator in einem Festbettreaktor oder mit einem gelösten Katalysator in einem Flüssigbettreaktor.

Die katalytische Umsetzung von Ammoniak, Kohlendioxid und Wasserstoff zu Ammoniumformiat oder zu Formamid und Ammoniumformiat kann kontinuierlich oder chargenweise durchgeführt werden, wobei ein kontinuierlicher Betrieb bevorzugt ist. Die katalytische Umsetzung wird vorzugsweise in einem Autoklav oder einem Druckreaktor durchgeführt. Ein Autoklav eignet sich für den Chargenbetrieb. Ein Druckreaktor eignet sich für den kontinuierlichen Betrieb. Die Konzentration an Katalysator, z.B. ein Ruthenium-Phosphin-Komplex als Katalysator, bei der Umsetzung kann z.B. im Bereich von 0,01 mmol-% bis 1,0 mol-%, bevorzugt von 0,1 mmol-% bis 0,5 mol-%, besonders bevorzugt 1,0 mmol-% bis 0,1 mol-%, bezogen auf die molare Menge an NH₃, liegen.

Die katalytische Umsetzung von Ammoniak, Kohlendioxid und Wasserstoff kann in Anwesenheit des Ruthenium-Phosphin-Komplexes als Katalysator oder in Anwesenheit des Ruthenium-Phosphin-Komplexes als Katalysator und einer Säure als Co-Katalysator durchgeführt werden. Die katalytische Umsetzung von Ammoniak, Kohlendioxid und Wasserstoff zu Ammoniumformiat oder zu Formamid und Ammoniumformiat erfolgt bevorzugt in Anwesenheit des Ruthenium-Phosphin-Komplexes und einer Säure. Die Säure dient dabei als Co-Katalysator zur Aktivierung des Katalysators und der Reaktanten und verbessert die Ausbeute der Reaktion.

Die Säure kann eine protische Säure (Brønsted-Säure) oder eine Lewis-Säure sein, wobei eine Lewis-Säure bevorzugt ist. Die Säure kann eine organische Säure oder eine anorganische Säure sein.

Beispiele für zweckmäßige Säuren, sind Organoaluminium-Verbindungen, wie z.B. Aluminiumtriflat (Aluminumtris(trifluoromethansulfonat), Al(OTf)₃ (Tf = -SO₂CF₃)) und Aluminiumtriacetat, Organobor-Verbindungen, wie z.B. Tris(pentafluorophenyl)boran, Bi(OTf)₃, 2,4,6-Trimethylbenzoesäure, Sulfonsäuren, wie z.B. p-Toluolsulfonsäure, Bis(trifluormethan)sulfonimid (HNTf₂), Scandiumverbindungen, wie z.B. Scandiumtriflat, perfluorierte Copolymere, die mindestens eine Sulfogruppe aufweisen, wie sie z.B. unter dem Handelsnamen Nafion^{®} NR50 erhältlich sind, oder Kombinationen davon.

Das Molverhältnis von Ruthenium-Phosphin-Komplex zu Säure, wenn eingesetzt, kann z.B. im Bereich von 1:800 bis 1:1, bevorzugt von 1:80 bis 1:5, besonders bevorzugt von 1:50 bis 1:10, liegen.

Die katalytische Umsetzung von Ammoniak, Kohlendioxid und Wasserstoff findet in wässriger Ammoniaklösung_statt, die neben Wasser gegebenenfalls mindestens ein organisches Lösungsmittel enthält. Bei der wässrigen Ammoniaklösung handelt es sich bevorzugt um eine wässrige oder wässrig-organische Lösung. Ammoniak löst sich sehr gut in Wasser. Kohlendioxid löst sich physikalisch nur geringfügig in Wasser. Die beiden Komponenten reagieren miteinander und dem Wasser unter Bildung eines komplexen Gemisches von gelösten Salzen, z.B. Ammoniumcarbonat, Ammoniumhydrogencarbonat, Ammoniumcarbamat und anderen Verbindungen.

Das wässrige Medium oder das wässrige Medium mit mindestens einem organischen Lösungsmittel kann Wasser oder vorzugsweise eine Mischung von Wasser und mindestens einem organischen Lösungsmittel sein. Das organische Lösungsmittel wird bevorzugt so gewählt, dass der eingesetzte Ruthenium-Phosphin-Komplex zumindest teilweise darin gelöst werden kann. Beispiele für geeignete organische Lösungsmittel sind zyklische und azyklische Ether, Ketone, Nitrile, Aromaten, Alkane, halogenierte Kohlenwasserstoffe und Alkohole. Konkrete Beispiele sind 1,4-Dioxan, Tetrahydrofuran, Acrylnitril, Acetonitril, Aceton und Toluol.

Die Zugabe von organischem Lösungsmittel erhöht die Löslichkeit des Katalysators und damit die Ausbeute an Ammoniumformiat, wodurch die Bildung von Formamid begünstigt wird. Die Bildung von Formamid ist hier aber nicht von prinzipieller Bedeutung, da Formamid insbesondere im nachfolgenden Schritt gebildet wird. Zudem befindet sich das bei der Umsetzung gebildete Ammoniumformiat nahezu ausschließlich in der wässrigen Phase, was die Auftrennung der Reaktionsprodukte erleichtert, wenn durch das organische Lösungsmittel auch eine organische Phase vorliegt (Zweiphasensystem).

Wenn mindestens ein organisches Lösungsmittel eingesetzt wird, kann das Verhältnis von Wasser und organischer Komponente in verschiedenen Verhältnissen variiert werden. In diesem Fall beträgt das Verhältnis von Wasser zu dem mindestens einen organischen Lösungsmittel, bezogen auf das Volumen, z.B. 100:1 bis 1:100, bevorzugt 1:10 bis 5:1, bevorzugter 1:5 bis 3:1, besonders bevorzugt 1:2 bis 2:1, z.B. 1:1.

In einer bevorzugten Ausführungsform wird der wässrigen Ammoniaklösung mindestens ein organisches Lösungsmittel zugesetzt, wobei das organische Lösungsmittel mit Wasser mischbar ist. Das organische Lösungsmittel ist dabei bevorzugt ein polares und aprotisches Lösungsmittel. Das organische Lösungsmittel ist bevorzugt ausgewählt aus der Gruppe bestehend aus Ethern, insbesondere zyklischen Ethern, wie z.B. Dioxan, insbesondere 1,4-Dioxan, und Tetrahydrofuran, Nitrilen, wie z.B. Acrylnitril und Acetonitril, und Ketonen, wie z.B. Aceton.

In einer alternativen bevorzugten Ausführungsform wird der wässrigen Ammoniaklösung mindestens ein organisches Lösungsmittel eingesetzt, das mit Wasser nicht mischbar ist, so dass sich ein Zweiphasensystem aus wässriger Phase und organischer Phase ausbildet. Hierfür eignen sich in der Regel unpolare organische Lösungsmittel. Beispiele für solche organischen Lösungsmittel sind Aromaten, wie z.B. Toluol, Ester, Ketone aber auch ionische Flüssigkeiten. Ionische Flüssigkeiten werden hier als organische Lösungsmittel angesehen. Bei Ausbildung eines Zweiphasensystems sollte dafür gesorgt werden, dass ein ausreichender Stoffaustausch zwischen den beiden Phasen gewährleistet ist, z.B. durch starkes Rühren und/oder Einsatz von einem Phasentransferkatalysator, wie z.B. Natriumdodecylsulfat.

Der Katalysator, insbesondere der Ruthenium-Phosphin-Komplex, liegt in der wässrigen Ammoniaklösung, die gegebenenfalls mindestens ein organisches Lösungsmittel enthält, bevorzugt zumindest teilweise in Lösung vor. Die katalytische Umsetzung von Ammoniak, Kohlendioxid und Wasserstoff ist vorzugsweise eine homogene katalytische Reaktion. Die homogene Katalyse kann mildere Reaktionsbedingungen und gegebenenfalls höhere Selektivitäten sowie höhere Wechselzahlen (Umsatzzahl TON ("turnover number") und/oder Umsatzfrequenz TOF ("turnover frequency")) ermöglichen.

Das Stoffmengenverhältnis der Edukte kann in breiten Bereichen variiert werden. Bezogen auf die Stöchiometrie der Reaktion ist das Stoffmengenverhältnis der Edukte NH₃:CO₂:H₂ 1:1:1. Abweichend von der Stöchiometrie werden CO₂ und H₂ bevorzugt im Überschuss zu NH₃ eingesetzt und H₂ im Überschuss zu COz, um die Ausbeute an Ammoniumformiat und/oder Formamid zu erhöhen. Beispielsweise kann für die Umsetzung von Ammoniak, Kohlendioxid und Wasserstoff ein Verhältnis von p(NH₃) : p(CO₂) : p(H₂) im Bereich von 1 : (1 bis 10) : (1 bis 20), bevorzugt im Bereich von 1 : (1,5 bis 10) : (2 bis 20), und besonders bevorzugt im Bereich von 1: (2 bis 5) : (3 bis 12), zweckmäßig sein, wobei p der Partialdruck der jeweiligen Edukts bei Raumtemperatur (23 °C) ist. Ein konkretes Beispiel für ein zweckmäßiges Verhältnis p(NH₃) : p(CO₂) : p(H₂) ist z.B. 8:32:80 (also 1:4:10).

Die katalytische Umsetzung von Ammoniak, Kohlendioxid und Wasserstoff erfolgt bevorzugt bei einer Temperatur im Bereich von 60 bis 180 °C und besonders bevorzugt im Bereich von 90 bis 110 °C.

Die katalytische Umsetzung von Ammoniak, Kohlendioxid und Wasserstoff erfolgt bevorzugt bei einem Druck im Bereich von 35 bar bis 210 bar, bevorzugter im Bereich von 40 bar bis 195 bar, besonders bevorzugt im Bereich von 80 bis 190 bar.

Die geeignete Reaktionsdauer für die katalytische Umsetzung von Ammoniak, Kohlendioxid und Wasserstoff kann in Abhängigkeit von den anderen Reaktionsparametern variieren. Zweckmäßig liegt die Reaktionsdauer der Umsetzung z.B. in einem Bereich von 1 Minute bis 24 Stunden, bevorzugt 30 Minuten bis 15 Stunden.

Das bei der katalytischen Umsetzung gemäß Gl. 8 gebildete Ammoniumformiat ist ein Zwischenprodukt, dessen Bildung durch das Wasser noch begünstigt wird. Die thermische Behandlung gemäß Gl. 9 führt zur Abspaltung von Wasser und zur Bildung des gewünschten Produkts Formamid. Die Reaktion kann bei Temperaturen von etwa 120 °C bereits anteilig stattfinden, so dass das Produkt Ammoniumformiat während der Umsetzung gemäß Gl. 8 bereits anteilig zum Formamid reagieren kann, allerdings nur in relativ geringfügigem Umfang. Bei höheren Temperaturen ergibt sich eine vollständigere Umwandlung. Bei niedrigeren Temperaturen kann auch praktisch kein Formamid gebildet werden, so dass nur Ammoniumformiat gebildet wird. Wasser begünstigt die Bildung des Ammoniumformiats durch ständigen Abtransport von Produkt aus der organischen Phase in die wässrige Phase, wenn ein Zweiphasensystem verwendet wird.

Findet die katalytische Umsetzung in Wasser oder einer Mischung von Wasser und einem mit Wasser mischbaren organischen Lösungsmittel statt, ist für die thermische Behandlung des Ammoniumformiats unter Bildung von Formamid das Wasser gewöhnlich ständig abzuführen. Ist eine mit Wasser nicht mischbare organische Phase vorhanden, ist gewöhnlich für die Auftrennung bzw. die Wiederaufarbeitung der organischen Phase zu sorgen. Organische Phasen begünstigen die Bildung von Formamid.

Bevorzugt erfolgt die katalytische Umsetzung in einem Zweiphasensystem aus wässriger Phase und organischer Phase. Als organische Phase eignet sich z.B. Toluol. Das gebildete Ammoniumformiat reichert sich nahezu ausschließlich in der wässrigen Phase an. Auch das Formamid, was bereits gebildet wurde, reichert sich größtenteils in der wässrigen Phase an. Die wässrige Phase kann abgetrennt und für die thermische Zersetzungsreaktion verwendet werden. Gegebenenfalls kann die wässrige Phase vor Durchführung der thermischen Zersetzungsreaktion zur Reinigung aufbereitet werden, z.B. durch Extraktion oder Dekantieren, um mitgeschlepptes organisches Lösungsmittel zu entfernen. Die Abtrennung der wässrigen Phase erfolgt vorzugsweise kontinuierlich während der katalytischen Umsetzung.

### Thermische Behandlung von Ammoniumformiat unter Bildung von Formamid gemäß Gl. 9

Nach der Bildung von Ammoniumformiat gemäß Gl. 8 wird das Ammoniumformiat einer Temperaturerhöhung unterworfen, wobei es unter Bildung von Formamid und Wasser zersetzt wird (thermische Abspaltung), wie in Gl. 9 dargestellt, so dass Formamid damit im Prinzip das einzige Reaktionsprodukt darstellt. Es handelt sich um eine allgemein bekannte Reaktion, für die kein Katalysator notwendig ist.

Die thermische Zersetzung von Ammoniumformiat zu Formamid und Wasser gemäß Gl. 9 erfolgt z.B. bei einer Temperatur im Bereich von 100°C bis 185 °C, bevorzugt mehr als 130 °C bis 185 °C und besonders bevorzugt bei 150 °C bis 180 °C. Die thermische Zersetzung wird bevorzugt bei Umgebungsdruck durchgeführt.

Für die thermische Zersetzung sind z.B. zwei prinzipielle Verfahrensführungen möglich. In einer Ausführungsform kann das gebildete Ammoniumformiat ohne vorherige Abtrennung in einer einstufigen Reaktion thermisch gespalten werden, wobei die Reaktionsmischung währenddessen oder danach durch Abdestillieren von Wasser aufkonzentriert wird.

In der alternativen und bevorzugten Ausführungsform erfolgt vor der Durchführung der thermischen Zersetzung eine Aufbereitung des aus der katalytischen Umsetzung erhaltenen Reaktionsgemisches, um störende Substanzen, wie z.B. Katalysator, Nebenprodukte und/oder Lösungsmittel abzutrennen. Dies erfolgt abhängig von der Art des erhaltenen Reaktionsgemisches über nachfolgend beschriebenen Varianten A und B, um eine abgetrennte bzw. gereinigte wässrige Phase zu erhalten.

Wenn die katalytische Umsetzung in einem Zweiphasensystem mit wässriger und organischer Phase durchgeführt wird, wird die wässrige Phase von der organischen Phase abgetrennt (Variante A). Wie vorstehend erläutert erfolgt die Abtrennung vorzugsweise kontinuierlich.

Wenn die katalytische Umsetzung in Wasser oder einer Mischung von Wasser und organischem Lösungsmittel durchgeführt wird (Einphasensystem), kann die wässrige Reaktionsmischung durch Extraktion mit einem mit Wasser nicht mischbaren organischen Lösungsmittel, z.B. Toluol, und abschließender Abtrennung der organischen Phase gereinigt werden (Variante B). Diese Zwischenextraktion kann ebenfalls kontinuierlich durchgeführt werden, um eine gereinigte wässrige Phase zu erhalten.

Die gemäß Variante A oder Variante B erhaltene abgetrennte bzw. gereinigte wässrige Phase, die das gebildete Ammoniumformiat und gegebenenfalls Formamid, das bei der Formiatsynthese bereits entstanden ist, enthält, wird der thermischen Zersetzung unterworfen, um das Ammoniumformiat in Formamid zu überführen. In der Regel wird von der Reaktionsmischung während und/oder nach der thermischen Zersetzungsreaktion Wasser abdestilliert.

In einer bevorzugten Ausführungsform erfolgt die thermische Zersetzung von Ammoniumformiat durch eine reaktive Destillation. Dabei wird die aus dem Verfahrensschritt a) erhaltene Reaktionsmischung oder vorzugsweise die gemäß Variante A oder Variante B erhaltene abgetrennte bzw. gereinigte wässrige Phase, die das gebildete Ammoniumformiat und gegebenenfalls Formamid, das bei der Formiatsynthese bereits entstanden ist, enthält, einer reaktiven Destillation zur thermischen Zersetzung des Ammoniumformiats unterworfen. In diesem Fall wird zuerst Wasser abdestilliert, wodurch das Gleichgewicht zum Formamid verschoben wird. Anschließend kann das Produkt Formamid rektifiziert werden, gegebenenfalls im Vakuum. Es ist aber auch denkbar, Formamid gar nicht zu destillieren und nach Abtrennung des Wassers den Rückstand ohne weitere Aufbereitung in der Harnstoffsynthese einzusetzen.

### Umsetzung von Formamid und Ammoniak unter Bildung von Harnstoff gemäß Gl. 6 oder Umsetzung von Formamid unter Bildung von Harnstoff

Das gebildete Formamid wird anschließend gemäß Schritt b) mit Ammoniak in Anwesenheit eines Katalysators umgesetzt, um Harnstoff zu erhalten (vgl. vorstehende Reaktionsgleichung Gl. 6). Alternativ wird das gebildete Formamid anschließend gemäß Schritt b) in Anwesenheit eines Katalysators umgesetzt, um Harnstoff zu erhalten (ohne Zugabe von Ammoniak). Die Umsetzung gemäß Reaktionsgleichung Gl. 6 oder gemäß der alternativen Verfahrensführung und die Aufarbeitung des erhaltenen Reaktionsgemisches erfolgt dabei genauso wie vorstehend in den alternativen Ausführungsformen auf Basis der mit CO₂ beladenen Methanolphase, so dass diesbezüglich auf das vorstehend Gesagte verwiesen wird.

### Ruthenium-Phosphin-Komplexe als Katalysatoren

Wie vorstehend erörtert ist ein Ruthenium-Katalysator, insbesondere ein Ruthenium-Phosphin-Komplex, ein geeigneter Katalysator für die folgenden Reaktionen, die in Anwesenheit eines Katalysators ausgeführt werden:
- Umsetzung von Formamid mit Ammoniak zur Bildung von Harnstoff und Wasserstoff (Gl. 6) oder Umsetzung von Formamid zur Bildung von Harnstoff und Wasserstoff ohne Zugabe von Ammoniak
- Umsetzung der mit CO₂ beladenen Methanolphase und dem Wasserstoff enthaltenden Strom zur Bildung von Methylformiat gemäß Variante a1) (Gl. 4)
- Umsetzung der mit CO₂ beladenen wässrigen Ammoniaklösung und dem Wasserstoff enthaltenden Strom zur Bildung von Ammoniumformiat oder zur Bildung von Ammoniumformiat und Formamid gemäß Variante a2) (Gl. 8).

Der Ruthenium-Phosphin-Komplex kann für eine, zwei oder alle drei Reaktionen verwendet. Es versteht sich, dass unterschiedliche oder gleiche Ruthenium-Phosphin-Komplexe für die Reaktionen eingesetzt werden können.

Der Ruthenium-Phosphin-Komplex weist einen oder mehrere Phosphin-Liganden auf. Bei dem Phosphin kann es sich um ein einfaches Phosphin (Monophosphin), eine Verbindung mit zwei Phosphingruppen (Diphosphin), eine Verbindung mit drei Phosphingruppen (Triphosphin) oder eine Verbindung mit mehr als drei Phosphingruppen handeln.

Bei den Phosphinen handelt es sich insbesondere um trivalente phosphororganische Verbindungen. Das Phosphin ist insbesondere ein tertiäres Phosphin oder weist zwei, drei oder mehr tertiäre Phosphingruppen auf. Bei dem Phosphin handelt es sich z.B. um eine Verbindung PR¹R²R³, worin R¹, R² und R³ unabhängig voneinander jeweils einen organischen Rest darstellen. Die Substituenten R¹, R² und R³ sind bevorzugt unabhängig voneinander jeweils substituiertes oder unsubstituiertes Alkyl, substituiertes oder unsubstituiertes Aryl oder substituiertes oder unsubstituiertes Heteroaryl.

Nachstehend sind geeignete und bevorzugte Beispiele für die Gruppen Alkyl, Aryl und Heteroaryl sowie geeignete Beispiele für Substituenten von entsprechenden substituierten Gruppen genannt, die als Beispiele für alle in der vorliegenden Anmeldung aufgeführten Bezugnahmen auf diese Gruppen bzw. substituierten Gruppen gelten, sofern nicht explizit ausgeschlossen. Die Beispiele für die Gruppen Alkyl, Aryl und Heteroaryl sind dabei auch Beispiele für diese Gruppen, wenn sie als Substituenten einer Gruppe vorliegen.

Alkyl schließt hier auch Cycloalkyl ein. Beispiele für Alkyl sind lineares und verzweigtes C₁-C₈-Alkyl, bevorzugt lineares und verzweigtes C₁-C₈-Alkyl, z.B. Methyl, Ethyl, n-Propyl, iso-Propyl oder Butyl, und C₃-C₈-Cycloalkyl.

Substituiertes Alkyl kann einen oder mehrere Substituenten aufweisen, z.B. Halogenid, wie Chlorid oder Fluorid, Aryl, Heteroaryl, Cycloalkyl, Alkoxy, z.B. C₁-C₈-Alkoxy, bevorzugt C₁-C₄-Alkoxy, oder Aryloxy. Bevorzugt ist unsubstituiertes Alkyl.

Beispiele für Aryl sind ausgewählt aus homoaromatischen Verbindungen mit einem Molekulargewicht unter 300 g/mol, vorzugsweise Phenyl, Biphenyl, Naphthalenyl, Anthracenyl und Phenanthrenyl.

Beispiele für Heteroaryl sind Pyridinyl, Pyrimidinyl, Pyrazinyl, Triazolyl, Pyridazinyl, 1,3,5-Triazinyl, Chinolinyl, Isochinolinyl, Chinoxalinyl, Imidazolyl, Pyrazolyl, Benzimidazolyl, Thiazolyl, Oxazolidinyl, Pyrrolyl, Carbazolyl, Indolyl und Isoindolyl, wobei das Heteroaryl mit der Phosphorgruppe des Phosphins über ein beliebiges Atom im Ring des ausgewählten Heteroaryls verbunden sein kann. Bevorzugte Beispiele sind Pyridinyl, Pyrimidinyl, Chinolinyl, Pyrazolyl, Triazolyl, Isochinolinyl, Imidazolyl und Oxazolidinyl, wobei das Heteroaryl mit der Phosphorgruppe des Phosphins über ein beliebiges Atom im Ring des ausgewählten Heteroaryls verbunden sein kann.

Substituiertes Aryl und substituiertes Heteroaryl können ein, zwei oder mehr Substituenten aufweisen. Beispiele für geeignete Substituenten für Aryl und Heteroaryl sind Alkyl, bevorzugt C₁-C₄-Alkyl, z.B. Methyl, Ethyl, n-Propyl, oder iso-Propyl, Alkoxy, z.B. Methoxy, Perfluoralkyl, z.B. -CF₃, Aryl, Heteroaryl, Cycloalkyl, Alkoxy, z.B. C₁-C₈-Alkoxy, bevorzugt C₁-C₄-Alkoxy, Aryloxy, Alkenyl, z.B. C₂-C₆-Alkenyl, bevorzugt C₃-C₆-Alkenyl, Silyl, Amin und Fluoren. Bevorzugt ist unsubstituiertes Aryl, insbesondere Phenyl, und unsubstituiertes Heteroaryl.

Gemäß einer bevorzugten Ausführungsform ist das Phosphin im Ruthenium-Phosphin-Komplex PR¹R²R³, worin R¹, R² und R³ unabhängig voneinander substituiertes oder unsubstituiertes Heteroaryl oder substituiertes oder unsubstituiertes Aryl, insbesondere Phenyl, sind, z.B. Tri(heteroaryl)phosphin oder Tri(aryl)phosphin, oder ein PR¹R²R³, worin R¹ Alkyl ist und R² und R³ unabhängig voneinander substituiertes oder unsubstituiertes Heteroaryl und/oder substituiertes oder unsubstituiertes Aryl, insbesondere Phenyl, sind, z.B. Di(heteroaryl)alkylphosphin oder Di(aryl)alkylphosphin.

Besonders bevorzugt ist das Phosphin im Ruthenium-Phosphin-Komplex eine Verbindung mit zwei Phosphingruppen (Diphosphin), eine Verbindung mit drei Phosphingruppen (Triphosphin) oder eine Verbindung mit mehr als drei Phosphingruppen, wobei das Phosphin besonders bevorzugt ein Triphosphin ist. Die Phosphine mit zwei oder mehr Phosphingruppen leiten sich bevorzugt von zwei oder mehr gleichen oder unterschiedlichen Phosphinen PR¹R²R³ wie vorstehend beschrieben ab, wobei mindestens ein Substituent der Phosphine mit einem oder mehreren anderen Substituenten der Phosphine unter Bildung einer gemeinsamen Gruppe, z.B. einer zwei-, drei- oder höherwertigen Alkylengruppe, als Brückeneinheit verknüpft ist. Die vorstehenden Angaben zu den Substituenten und bevorzugten Substituenten bzw. Phosphinen gelten analog für die Verbindungen mit mehr als einer Phosphingruppe.

Gemäß einer bevorzugten Ausführungsform enthält der Ruthenium-Phosphin-Komplex mehr als eine Phosphingruppe, d.h. dass in der Koordinationssphäre des Rutheniums als Liganden zwei oder mehr Monophosphine, mindestens ein Diphosphin oder Triphosphin oder eine Verbindung mit mehr als drei Phosphingruppen vorhanden sind.

Die Bindungen zwischen dem Ruthenium und der Phosphingruppe werden zumindest zeitweise während der Reaktion gebildet, z.B. eine kovalente oder koordinative Bindung. Es sollte angemerkt werden, dass bei der erfindungsgemäßen Umsetzung in Anwesenheit des Ruthenium-Phosphin-Komplex nicht alle Phosphine bzw. Phosphingruppen in der Reaktionsmischung notwendigerweise an das Ruthenium gebunden sind. Tatsächlich kann das Phosphin im Überschuss verwendet, so dass auch nicht gebundene Phosphine bzw. Phosphingruppen in der Reaktionsmischung vorhanden sein können. Insbesondere wenn Verbindungen mit mehr als drei Phosphingruppen verwendet werden, sind in der Regel nicht alle Phosphoratome katalytisch an der Reaktion beteiligt; dennoch sind diese Verbindungen auch innerhalb der vorliegenden Erfindung bevorzugte Verbindungen.

Besonders bevorzugt sind Ruthenium-Triphosphin-Komplexe, wobei bei dem Triphosphin die Brückeneinheit zwischen den Phosphoratomen eine Alkyl- bzw. Alkyleneinheit ist, während die weiteren Liganden am Phosphor substituiertes oder unsubstituiertes Aryl oder substituiertes oder unsubstituiertes Heteroaryl sind.

Gemäß einer bevorzugten Ausführungsform umfasst der Ruthenium-Triphosphin-Komplex ein Triphosphin der allgemeinen Formel I 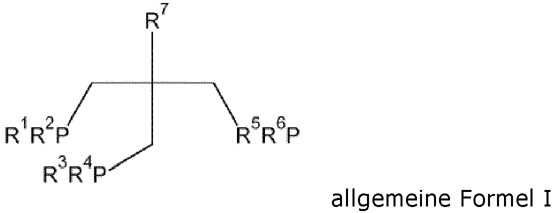 wobei R¹ bis R⁶ unabhängig voneinander substituiertes oder unsubstituiertes Aryl oder substituiertes oder unsubstituiertes Heteroaryl, bevorzugt substituiertes oder unsubstituiertes Aryl sind und R⁷ Wasserstoff oder eine organische Komponente, vorzugsweise Alkyl, Cycloalkyl oder Aryl ist. Beispiele für geeignete Substituenten für Aryl und Heteroaryl sind vorstehend genannt, bevorzugt sind Alkyl, insbesondere Methyl, Ethyl, n-Propyl, iso-Propyl, Alkoxy, wie Methoxy, oder Perfluoralkyl, wie -CF₃. Das substituierte oder unsubstituierte Aryl ist bevorzugt unsubstituiertes Aryl, insbesondere Phenyl. Das substituierte oder unsubstituierte Heteroaryl ist bevorzugt unsubstituiertes Heteroaryl.

Die Substituenten R¹ bis R⁶ können gleich oder verschieden sein, wobei sie bevorzugt gleich sind. Besonders bevorzugt ist R¹ bis R⁶ unabhängig voneinander substituiertes oder unsubstituiertes Phenyl. Das substituierte Aryl, insbesondere substituiertes Phenyl, kann ein, zwei oder mehr Substituenten aufweisen, z.B. in ortho- und/oder para-Stellung. Beispiele für geeignete Substituenten sind vorstehend genannt, bevorzugt sind Alkyl, insbesondere Methyl, Ethyl, n-Propyl, iso-Propyl, Alkoxy, wie Methoxy, oder Perfluoralkyl, wie -CF₃. Besonders bevorzugt ist R⁷ Alkyl, bevorzugter Methyl oder Ethyl, insbesondere Methyl.

Ein besonders bevorzugter Phosphin-Ligand für den Ruthenium-Phosphin-Komplex ist 1,1,1-Tris(diphenylphosphinomethyl)ethan (Triphos), welches folgende Struktur aufweist:

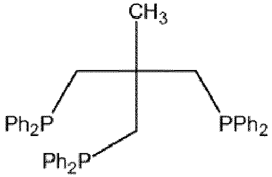

Der Ruthenium-Phosphin-Komplex kann neben dem oder den vorstehend genannten Phosphin-Liganden ein oder mehrere weitere Liganden (Nicht-Phosphin-Liganden) aufweisen, wie z.B. Carbene, Amine, Amide, Phosphite, Phosphoamidite, phosphorhaltige Ether oder Ester, Sulfide, Trimethylenmethan, Cyclopentadienyl, Allyl, Methylallyl, Ethylen, Cyclooctadien, Acetylacetonat, Acetat, Hydrid, Halogenid, wie z.B. Chlorid, Phenolat oder CO, insbesondere wenn der Ruthenium-Phosphin-Komplex ein vorstehend beschriebenes Diphosphin, Triphosphin oder eine Verbindung mit mehr als drei Phosphingruppen aufweist.

Der eine oder die mehreren weiteren Liganden sind bevorzugt ausgewählt aus Trimethylenmethan, Cyclopentadienyl, Allyl, Methylallyl, Ethylen, Cyclooctadien, Acetylacetonat, Acetat, Hydrid, Halogenid, Phenolat, CO oder einer Kombination davon, wobei Trimethylenmethan (tmm) besonders bevorzugt ist. Diese Liganden weisen eine relativ labile Bindung zu Ruthenium auf, so dass sie während der katalytischen Reaktionssequenz leicht durch Reaktantenspezies mit oder ohne Beihilfe des Aktivators/Co-Katalysators substituiert werden können. Ferner kann mit diesen Liganden eine Katalysatorvorstufe stabilisiert werden.

In einer bevorzugten Ausführungsform weist der Ruthenium-Phosphin-Komplex die folgende allgemeine Formel II auf:

(A)Ru(L)₃ allgemeine Formel II

worin A ein Triphosphin der allgemeinen Formel I wie vorstehend definiert ist und L jeweils unabhängig voneinander einzähnige Liganden sind, wobei zwei einzähnige Liganden L durch einen zweizähnigen Liganden ersetzt sein können oder drei einzähnige Liganden L durch einen dreizähnigen Liganden ersetzt sein können. Beispiele für die ein-, zwei- oder dreizähnigen Liganden L sind die vorstehend genannten weiteren Liganden (Nicht-Phosphin-Liganden), wobei sie bevorzugt ausgewählt sind aus Trimethylenmethan, Cyclopentadienyl, Allyl, Methylallyl, Ethylen, Cyclooctadien, Acetylacetonat, Acetat, Hydrid, Halogenid, Phenolat, CO oder einer Kombination davon, wobei Trimethylenmethan (tmm) besonders bevorzugt ist. Beispielsweise ist der Ligand tmm ein dreizähniger Ligand.

Ein besonders bevorzugter Ruthenium-Triphosphin-Komplex weist die folgende Struktur auf: 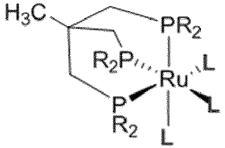 wobei die Substituenten R jeweils unabhängig voneinander substituiertes oder unsubstituiertes Aryl oder substituiertes oder unsubstituiertes Heteroaryl, bevorzugt substituiertes oder unsubstituiertes Aryl, sind und die Substituenten L jeweils unabhängig voneinander einzähnige Liganden sind, wobei zwei einzähnige Liganden L durch einen zweizähnigen Liganden ersetzt sein können oder drei einzähnige Liganden L durch einen dreizähnigen Liganden ersetzt sein können. Beispiele für geeignete Substituenten für Aryl und Heteroaryl sind vorstehend genannt, bevorzugt sind Alkyl, insbesondere Methyl, Ethyl, n-Propyl, iso-Propyl, Alkoxy, z.B. Methoxy und Perfluoralkyl, wie -CF₃. Das substituierte oder unsubstituierte Aryl ist bevorzugt unsubstituiertes Aryl, insbesondere Phenyl. Das substituierte oder unsubstituierte Heteroaryl ist bevorzugt unsubstituiertes Heteroaryl.

Die Substituenten R können gleich oder verschieden sein, wobei sie bevorzugt gleich sind. Besonders bevorzugt ist R unabhängig voneinander substituiertes oder unsubstituiertes Phenyl. Das substituierte Phenyl kann ein, zwei oder mehr Substituenten aufweisen, insbesondere in ortho- und/oder para-Stellung. Beispiele für geeignete Substituenten sind vorstehend genannt, bevorzugt sind Alkyl, insbesondere Methyl, Ethyl, n-Propyl, iso-Propyl, Alkoxy, z.B. Methoxy, und Perfluoralkyl, wie -CF₃. Der Triphosphin-Ligand ist besonders bevorzugt Triphos.

Beispiele für die ein-, zwei- oder dreizähnigen Liganden L sind die vorstehend genannten weiteren Liganden (Nicht-Phosphin-Liganden), wobei sie bevorzugt ausgewählt sind aus Trimethylenmethan, Cyclopentadienyl, Allyl, Methylallyl, Ethylen, Cyclooctadien, Acetylacetonat, Acetat, Hydrid, Halogenid, Phenolat, CO oder einer Kombination davon, wobei Trimethylenmethan (tmm) besonders bevorzugt ist.

Ein besonders bevorzugter Ruthenium-Phosphin-Komplex ist [Ru(Triphos)(tmm)] mit folgender Strukturformel:

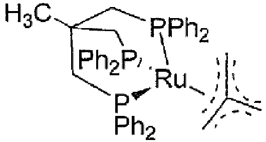

Die vorstehend genannten Ruthenium-Phosphin-Komplexe sind bekannt und können vom Fachmann nach bekannten Methoden hergestellt werden bzw. sind im Handel erhältlich. [Ru(Triphos)(tmm)] wird z.B. in T. vom Stein et al., ChemCatChem 2013, 5, 439-441; beschrieben.

Der Ruthenium-Phosphin-Komplex kann bei der katalytischen Umsetzung gemäß der Gl. 4, Gl. 6 oder Gl. 8 wie vorstehend beschrieben unabhängig voneinander als homogener Katalysator oder als immobilisierter Katalysator eingesetzt werden. Die katalytische Umsetzung mit dem Ruthenium-Phosphin-Komplex kann homogen oder heterogen durchgeführt werden, z.B. mit einem immobilisierten Katalysator in einem Festbettreaktor oder mit einem gelösten Katalysator in einem Flüssigbettreaktor.

Die katalytische Umsetzung gemäß der Gl. 4, Gl. 6 oder Gl. 8 wie vorstehend beschrieben unabhängig voneinander kann kontinuierlich oder chargenweise durchgeführt werden, wobei ein kontinuierlicher Betrieb bevorzugt ist. Die katalytische Umsetzung wird vorzugsweise in einem Autoklav oder einem Druckreaktor durchgeführt. Ein Autoklav eignet sich für den Chargenbetrieb. Ein Druckreaktor eignet sich für den kontinuierlichen Betrieb.

### Integration des Verfahrens in eine Ammoniak-Anlage

Wie vorstehend erläutert, kann neben der Standardroute für die Bereitstellung des Synthesegases für die Ammoniaksynthese über die Dampfreformierung alternativ oder zusätzlich (als Beimischung zu dem Synthesegas aus der Dampfreformierung) ein gegebenenfalls aufbereitetes Gas ausgewählt aus einem Koksofengas, einem Hochofengas oder einem Abgas von Zementwerken als Synthesegas verwendet werden, wobei ein Synthesegas für die Ammoniaksynthese aber bevorzugt ist

Wie bereits vorstehend ausgeführt, ist das erfindungsgemäße Verfahren in einer bevorzugten Ausführungsform mit einer üblichen Ammoniak-Synthese gekoppelt. Die Ammoniak-Synthese umfasst dabei im Allgemeinen in dieser Reihenfolge die Herstellung eines Synthesegases durch Dampfreformierung, insbesondere umfassend die Umsetzung in einem Primärreformer und einem nachgeschalteten Sekundärreformer, Wassergas-Shift-Reaktion, in der Regel zweistufig als Hochtemperatur- und Niedertemperatur-Shiftstufe ausgeführt, Gaswäsche des gebildeten Synthesegases mit einer Waschflüssigkeit zur Entfernung von COz, Methanisierung des gereinigten Synthesegases und die Herstellung von Ammoniak mit dem Synthesegas in herkömmlicher Weise. Nach der Methanisierung wird das Synthesegas gewöhnlich für die anschließende Ammoniak-Synthese verdichtet.

Bei dem erfindungsgemäßen Verfahren zur Herstellung des Harnstoffs wird dabei die Ammoniaksynthese als Quelle für die Einsatzstoffe Kohlendioxid, Wasserstoff und Ammoniak verwendet. Wie vorstehend ausgeführt, wird die mit COz beladene Waschflüssigkeit, die aus der Gaswäsche des Synthesegases erhalten wird, als Quelle für Kohlendioxid genutzt.

Als Quelle für Wasserstoff in Form des Wasserstoff enthaltenden Stroms kann ein Teilstrom des durch die Gaswäsche gereinigten Synthesegases (nach der Gaswäsche) verwendet werden, wobei der Teilstrom vor der gegebenenfalls durchgeführten Methanisierung des Synthesegases entnommen werden kann, ein Teilstrom des Synthesegases vor der Gaswäsche (Rohsynthesegas), ein aus der Aufarbeitung der Produkte der Harnstoff-Synthese erhaltener Wasserstoff oder eine Kombination davon verwendet werden. Als Quelle für Wasserstoff kann gegebenenfalls alternativ oder zusätzlich aus der Aufarbeitung der Produkte der Ammoniak-Synthese erhaltener Wasserstoff verwendet werden.

Die vorstehend angeführte Entnahme des Synthesegases vor der Gaswäsche ist nur dann zweckmäßig, wenn der Druck bei der Formamidsynthese niedrig ist, insbesondere weniger als etwa 35 bar. In anderen Fällen muss ein hochkomprimiertes Gas entnommen werden, welches zwangsläufig eine oder mehrere Kompressorstufen/Kompressionstufen durchlaufen hat. In diesem Fall werden keine Wasserstoffverluste erwartet, da die Spuren von CO/CO₂ im Synthesegas in der Formamidsynthese umgesetzt werden. Für die Entfernung des Rest-COz aus dem Rezyklierungsstrom aus der Formamidsynthese bieten sich drei Lösungsmöglichkeiten (oder deren Kombination) an:
1. Der Wasserstoff enthaltende Teilstrom nach der Gaswäsche wird bevorzugt vor einer gegebenenfalls durchgeführten Methanisierung des Synthesegases entnommen.
2. Rückführung des Off-Gases aus der Formamidsynthese in die Wäsche. In diesem Fall sind die Betriebskosten für Kompression wahrscheinlich höher, da zusätzlicher Gasstrom zu komprimieren ist.
3. Zusätzliche Wäsche, die das Off-Gas von COz befreien soll unter Verwendung von gleichem Waschmittel und Vereinigung der Stoffströme von beiden Wäschen. Dieser von COz befreite Stoffstrom kann ggf. mithilfe des wasserstoffhaltigen Stroms aus der Harnstoff-Anlage zu dem Synthesegas aufbereitet werden.

Wie vorstehend beschrieben, ist die Möglichkeit der Nutzung des bei der Harnstoff-Synthese erzeugten Wasserstoffs als Quelle für Wasserstoff ein besonderer Vorteil der Erfindung. Die Ausführungsform, bei der für den eingesetzten Wasserstoff zumindest teilweise ein Wasserstoff enthaltender Teilstrom des Synthesegases vor der Gaswäsche verwendet wird, bezieht sich auf ein Synthesegas, das nicht der Gaswäsche unterworfen wurde. Der Wasserstoff enthaltende Teilstrom des Synthesegases vor der Gaswäsche ist ein auch CO und COz enthaltendes Rohsynthesegas. Bei dessen Einsatz entfällt die Reinigung eines Teils des Rohsynthesegases und die CO₂-Wäsche bzw. CO₂-Bindung kann um diesen Betrag entlastet werden. Die Voraussetzungen hierfür wurden vorstehend diskutiert. Damit ohne Wäsche und anschließende Kompression entnommen werden kann, soll der Druck der Formamidsynthese unter 35 bar liegen.

Als Quelle für Ammoniak in Form des Ammoniak enthaltenden Stroms und/oder der wässrigen Ammoniaklösung wird das aus der Ammoniak-Synthese gewonnene Ammoniak verwendet. Als Quelle für Ammoniak können gegebenenfalls zusätzlich aus der Aufarbeitung der Produkte der Harnstoff-Synthese erhaltenes Ammoniak verwendet werden oder auch externe Quellen, z.B. aus einer weiteren Ammoniak-Anlage.

Die geringfügigen Veränderungen der klassischen Ammoniak-Prozessführung, die für die erfindungsgemäße katalytische Harnstoffsynthese nötig sind, werden im Folgenden näher erläutert. Bis zur COz-Wäsche verläuft der Prozess identisch zu der klassischen Ammoniak-Synthese. Erst bei der COz-Wäsche kann sich die Prozessführung ändern. Die mit physikalisch absorbiertem CO₂ beladene Waschflüssigkeit auf Basis von Methanol (z.B. nach dem Rectisol-Verfahren) oder die mit chemisch absorbiertem COz beladene Waschflüssigkeit auf Basis von wässrigem Ammoniak wird, ohne diese zu regenerieren, in dem Umfang zu der Formamid-Synthese geleitet, welcher für die Harnstoff-Synthese nötig ist. Die Regeneration des Lösungsmittels findet im Fall von Methanol während der Reaktion statt. Überschüssiges und während der Reaktion freigesetztes MeOH (Gl. 4 und Gl. 5) wird aufbereitet und zurückgeführt. Im Fall von wässrigem Ammoniak wird die beladene Lösung zumindest größtenteils verbraucht.

Das durch die Gaswäsche gereinigte Synthesegas und/oder das Rohsynthesegas kann aufgeteilt und ein Teilstrom davon abgezweigt und als Quelle für Wasserstoff in die Formamid-Synthese geleitet werden (vergleiche die vorstehenden Anmerkungen hierzu). Alternativ oder zusätzlich kann der bei der Harnstoffsynthese gebildete Wasserstoff als Quelle für Wasserstoff genutzt werden. Besonders bevorzugt wird der bei der Harnstoffsynthese gebildete Wasserstoff genutzt, die gemäß der Stöchiometrie ausreichend Wasserstoff für die Formamid-Synthese produziert, wobei zusätzlich ein Teilstrom des gereinigten Synthesegases und/oder des Synthesegases genutzt werden kann, solange die Harnstoff-Synthese nicht läuft oder nicht stationär läuft und um möglicherweise produktionsbedingte Wasserstoffverluste bei der Harnstoffsynthese auszugleichen. Die Aufteilung des gereinigten Synthesegases kann, je nach gewählten Parametern in der Formamid-Synthese, z.B. noch vor der Methanisierung oder nach der Verdichtung des Synthesegases erfolgen. Das stark wasserstoffhaltige Gas aus der H₂-Rückgewinnung nach der Ammoniak-Synthese kann ebenfalls in die Formamid-Synthese geleitet werden. Anschließend wird synthetisiertes Ammoniak für die Reaktion (2) in die Formamid-Synthese geleitet oder gegebenenfalls für die Herstellung von wässrigem Ammoniak aber mit demselben Zweck genutzt.

Zu den "Produktströmen" der Formamid-Synthese zählen bei der Variante auf Basis einer Methanolphase außer Formamid selbst das Wasser, Stickstoff aus dem Synthesegas und Methanol. Das Wasser wird abgetrennt und in eine Wasseraufbereitung geleitet oder entsorgt.

Das Methanol kann nach einer Wiederaufbereitung z.B. für die Wäsche (Rectisol) wiederverwendet werden. Die Aufbereitung des Methanols ist erforderlich, um es von Wasser zu befreien, da das Wasser auch im Rohsynthesegas vorhanden ist und mitgenommen wird. Zudem wird Wasser in der Reaktion gebildet. Die Wiederaufbereitung des Methanols zur Entfernung von Wasser ist erforderlich, da das Wasser bei der Wiederverwendung des Methanols in der Wäsche stört. Bei hohem Wassergehalt kann ein Wasser/Methanol-Gemisch je nach eingesetzter Temperatur sogar einfrieren. Der Stickstoff kann z.B. mit dem Wasserstoff aus der Harnstoff-Synthese kombiniert und zum Synthesegas aufbereitet werden. Dieser Anteil des Synthesegases entspricht grundsätzlich der Menge, die aus dem Synthesegas für die Formamid-Synthese entnommen wurde. Dieser Anteil des Synthesegases mit oder ohne zusätzliche Aufbereitung kann mit dem restlichen Synthesegas vor der Methanisierung vereinigt werden, da dieses Gas gegebenenfalls bei niedrigerem Druck einfällt und komprimiert werden muss. Die genaue Position der Methanisierung und der Wiedereinführung des Teiles des Synthesegases hängt von dem Druck der Formamid-Synthese und der Harnstoffsynthese ab, der theoretisch beliebig sein kann. Es wird auf die vorstehenden Ausführungen zur Entnahme des Synthesegases verwiesen.

Nachfolgend wird die Erfindung anhand von Ausführungsbeispielen beschrieben, die anhand der Abbildungen näher erläutert werden. Die konkreten Ausführungsbeispiele sollen den Umfang der beanspruchten Erfindung in keiner Weise beschränken. Es zeigen:
- Fig. 1: ein Blockfließbild eines konkreten Beispiels für ein erfindungsgemäßes Verfahren zur Herstellung von Harnstoff, gekoppelt mit einer Ammoniak-Synthese;
- Fig. 2: ein Reaktionsschema der Variante der Formamid-Synthese über die Methanolroute (Methylformiat);
- Fig. 3: ein Reaktionsschema der Variante der Formamid-Synthese über die wässrige Route (Ammoniumformiat);
- Fig. 4: ein Blockfließbild eines Beispiels für die Aufbereitung des Harnstoffs.

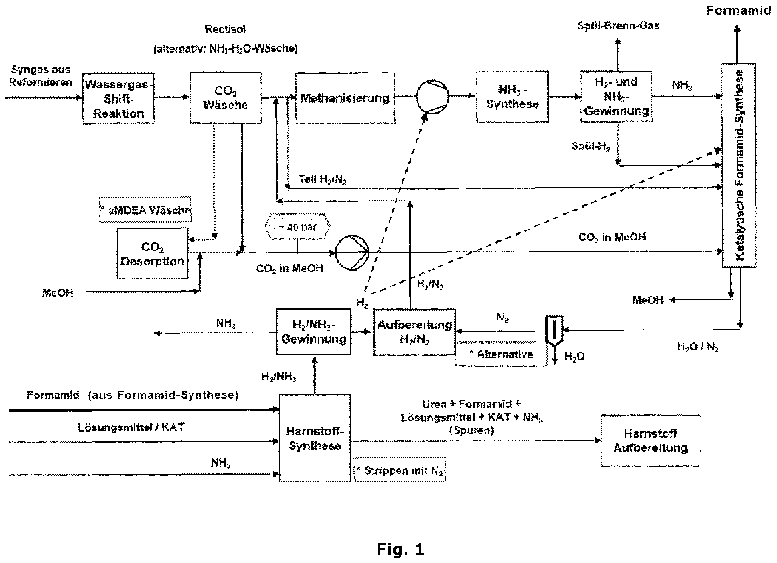

Fig. 1 zeigt ein Blockfließbild für ein Beispiel eines erfindungsgemäßen Verfahrens zur Herstellung des Harnstoffs, das mit einer Ammoniak-Synthese gekoppelt ist. Ein Synthesegas, das hier aus einer Dampfreformierung stammt und für die Ammoniaksynthese vorgesehen ist, enthält Wasserstoff, Stickstoff und Kohlenmonoxid. Das Synthesegas wird einer Wassergas-Shift-Reaktion (Wassergas-Konvertierungsreaktion) unterzogen, um Kohlenmonoxid in Kohlendioxid zu überführen. Im Anschluss wird eine Gaswäsche durchgeführt, um Kohlendioxid aus dem Synthesegas zu entfernen. Die Gaswäsche erfolgt mittels einer Waschflüssigkeit, wobei die Waschflüssigkeit während der Gaswäsche mit chemisch und/oder physikalisch gebundenem Kohlendioxid beladen wird.

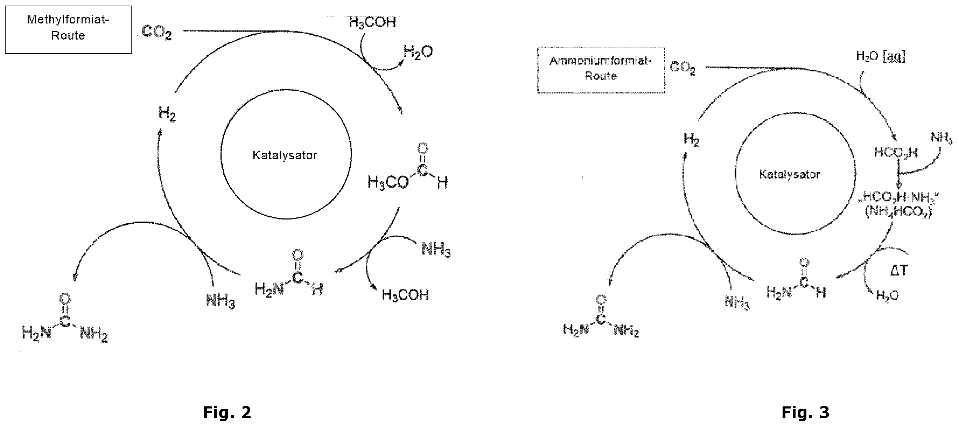

In diesem Ausführungsbeispiel erfolgt die Gaswäsche mit einer Waschflüssigkeit, die Methanol ist (Rectisol). Das Methanol wird während der Gaswäsche mit Kohlendioxid beladen. Der resultierende Strom wird als COz enthaltender Strom für die katalytische Formamid-Synthese verwendet (Zwischenprodukt Methylformiat). Die katalytische Formamid-Synthese ist in Fig. 1 nur schematisch dargestellt, sie kann wie beschrieben einstufig oder zweistufig (mit oder ohne Isolierung der Zwischenstufe) unter Bildung von Methylformiat als Zwischenprodukt und dann Bildung des Formamids als Endprodukt durchgeführt werden. Ein Reaktionsschema für diese Variante ist in Fig. 2 gezeigt. Das gebildete Formamid wird als Edukt zur Harnstoff-Synthese geleitet.

Eine alternative Ausführungsform ist mit gestrichelten Linien dargestellt. Hierbei erfolgt die Gaswäsche mit einer wässrigen Lösung von Methyldiethanolamin mit Piperazin als Waschflüssigkeit (aMDEA-Wäsche). Es kann alternativ jede konventionelle Waschflüssigkeit eingesetzt werden, welche dem Fachmann bekannt ist, z.B. wässriges Kaliumcarbonat (Benfield-Wäsche). Aus der mit Kohlendioxid beladenen Waschflüssigkeit wird mittels COz-Desorption COz desorbiert und das freigesetzte COz in Methanol absorbiert, um eine mit COz beladene Methanolphase zu erhalten. Der resultierende Strom wird analog wie bei der oben beschriebenen Rectisol-Wäsche als COz enthaltender Strom für die katalytische Formamid-Synthese verwendet, wofür der Druck des COz vor oder nach der Absorption ins MeOH erhöht werden muss.

In einer weiteren, nicht gezeigten alternativen Ausführungsform erfolgt die Gaswäsche mit einer wässrigen Ammoniaklösung (NH₃-H₂O-Wäsche). Dabei wird COz physikalisch und in Form von Carbonaten und Carbamat in der Waschflüssigkeit gebunden und direkt als COz enthaltender Strom für die katalytische Formamid-Synthese verwendet (Zwischenprodukt Ammoniumformiat). Bei dieser Variante kann die katalytische Formamid-Synthese wie beschrieben einstufig oder zweistufig (mit oder ohne Isolierung der Zwischenstufe) unter Bildung von Ammoniumformiat als Zwischenprodukt und anschließender Bildung des Formamids als Endprodukt durchgeführt werden. Ein Reaktionsschema für die alternative Variante auf Basis der wässrigen Ammoniaklösung mit der anschließenden Harnstoff-Synthese ist in Fig. 3 gezeigt.

Als Wasserstoff enthaltender Strom kann ein Teilstrom des Synthesegases, enthaltend Wasserstoff und Stickstoff, vor oder nach der Gaswäsche abgezweigt und für die Bildung von Formamid verwendet werden (in Fig. 1 nach der Gaswäsche und vor der Methanisierung). Das übrige Synthesegas wird einer Methanisierung unterzogen, um durch Methanbildung weiteres Kohlenmonoxid und/oder Kohlendioxid aus dem Synthesegas zu entfernen. Dann wird das Synthesegas nach Verdichtung für die Ammoniaksynthese verwendet. Das aus der Ammoniaksynthese gewonnene Reaktionsgemisch wird zur NH₃- und H₂-Rückgewinnung aufgearbeitet. Das erhaltene Ammoniak und der erhaltene Wasserstoff können auch für die Formamid-Synthese eingesetzt werden. Der erhaltene Wasserstoff kann zurück in die NH₃-Synthese geführt werden oder als Make-Up-Strom für die Formamid-Synthese verwendet werden, wenn geringe Mengen H₂ in der Formamid-Synthese verloren gehen. Andere Varianten der Synthesegas-Entnahme wurden vorstehend beschrieben.

Wie vorstehend erläutert, wird bevorzugt der Wasserstoff aus der Harnstoff-Synthese als Wasserstoff enthaltender Strom für die Bildung von Formamid verwendet (in Fig. 1 als gestrichelter Pfeil gezeigt) und der Teilstrom des Synthesegases kann ergänzend genutzt werden, wenn die Harnstoffsynthese nicht läuft und/oder um eventuelle Wasserstoffverluste bei der Harnstoff-Synthese auszugleichen.

Das bei der Umsetzung von Methylformiat mit Ammoniak zur Bildung von Formamid ebenfalls gebildete Methanol wird für die Waschflüssigkeit oder die Methanolphase in dem Verfahren wiederverwendet. Es wird zusammen mit überschüssigem MeOH aus der Waschflüssigkeit wiederaufbereitet und in die Wäsche zurückgeführt.

Konkrete Beispiele für die Umsetzungen sind weiter unten angeführt.

Nach der Harnstoff-Synthese erfolgt die Aufbereitung des gebildeten Gemisches enthaltend Harnstoff (Urea), Formamid, Lösungsmittel, Katalysator (KAT), Ammoniak (NH₃) und Wasserstoff (H₂). Einzelheiten zur möglichen Aufbereitung des bei der Harnstoff-Synthese erhaltenen Produkts sind nachstehend in Fig. 4 erläutert. Aus der Aufbereitung der Produkte der Harnstoff-Synthese erhaltener Wasserstoff wird nach H₂- und NH₃-Rückgewinnung und gegebenenfalls Aufbereitung von H₂/N₂ für das Synthesegas verwendet. Mit anderen Worten kann der bei der Herstellung des Harnstoffs aus Formamid und Ammoniak gebildete Wasserstoff in den Prozess zurückgeführt werden. Der bei der H₂-/NH₃-Gewinnung erhaltene Ammoniak kann gleichfalls im Prozess an vielen Stellen wiederverwendet werden.

Fig. 2 zeigt ein schematisches Reaktionsschema der Variante der Formamid-Synthese über die "Methanolroute" zum Formamid über Methylformiat und die Umsetzung des Formamids zum Harnstoff entsprechend den Gleichungen Gl. 4, Gl. 5 und Gl. 6 mit dem Edukt- und Produktkreislauf. Die gezeigten Reaktionen sind vorstehend im Detail beschrieben.

Fig. 3 zeigt ein schematisches Reaktionsschema der Variante der Formamid-Synthese über die "wässrige Route" zum Formamid über Ammoniumformiat und die Umsetzung des Formamids zum Harnstoff entsprechend den Gleichungen Gl. 8, Gl. 9 und Gl. 6 mit dem Edukt- und Produktkreislauf. Die gezeigten Reaktionen sind vorstehend im Detail beschrieben.

Fig. 4 zeigt ein Blockfließbild eines Beispiels zur möglichen Aufbereitung des bei der Harnstoff-Synthese erhaltenen Produkts. In Fig. 4 sind für die Harnstoffsynthese beispielhaft ein Druck von 8 bar und eine Temperatur von 150 °C angegeben. Die Temperatur kann deutlich geringer sein, aber auch der Druck kann deutlich höher sein. Nach Herstellung des Harnstoffs kann die Temperatur eventuell gesenkt werden (Wärmeintegration), ansonsten ergibt sich ein Hochdruckflash. Eine hohe Temperatur ist aber vorteilhaft, da mehr Wasserstoff und Ammoniak abgetrennt werden, auch bei hohem Druck. Eine Druckveränderung soll möglichst vermieden werden. Wie oben beschrieben werden Wasserstoff und Ammoniak aus der Mutterlauge als Gas abgezogen, optional unter Verwendung von Stickstoff als Strippgas. Dieses Gasgemisch wird auf ähnliche Art und Weise wie es in der Ammoniaksynthese üblich ist aufbereitet. Der flüssige Reaktionsrückstand, enthaltend Harnstoff, Lösungsmittel, Katalysator, Formamid und Spuren von Ammoniak, wird gemäß üblichen Verfahren weiter aufbereitet. Die Mutterlauge wird bis auf etwa -30 °C abgekühlt und einer Filtration unterzogen. Bei der Abkühlung fällt der überwiegende Anteil des Harnstoffes aus der Mutterlösung aus. Der Rückstand enthält Harnstoff und Spuren von Lösungsmittel, Formamid und Katalysator. Diese werden durch Nachwaschen mit frischem, gegebenenfalls kaltem Lösungsmittel entfernt und der Rückstand, enthaltend Harnstoff und Spuren des Lösungsmittels, einer Granulation unterzogen, um den Harnstoff zu erhalten.

Alternativ kann das Filtrat nach der Abtrennung vom Harnstoff mit frischen Komponenten NH₃ und Formamid versetzt (in Mengen, die verbraucht wurden) und direkt in die Harnstoff-Synthese zurückgeführt werden. Am besten wird das Lösungsmittel, das beim Nachwaschen vom abfiltrierten Harnstoff zurückbleibt, mit dem Filtrat vereinigt und eingeengt, um Verluste an Katalysator zu vermeiden. Das abdestillierte Lösungsmittel kann für die Nachwäsche wiederverwendet werden und die aufkonzentrierte Lösung kann zurück in die Harnstoffsynthese geführt werden.

Die Aufbereitung des Rückstandes (Waschen mit frischem kaltem Lösungsmittel, Abtrennung des Katalysators usw.) kann alternativ in einem separaten, auch geschlossenen Kreislauf erfolgen. Die zurückgewonnenen Komponenten können in zeitlichen Intervallen den Hauptströmen (z.B. Harnstoff, Katalysator oder Formamid) beigemischt werden (gestrichelter Pfeil in Fig. 4). Die zum Waschen eingesetzte Waschlösung wird mit dem Filtrat vereint. Die erhaltene Mischung enthält Lösungsmittel, Katalysator, Formamid und Spuren von Harnstoff. Die Mischung wird eingedampft, optional im Vakuum. Das abdestillierte Lösungsmittel und die aufkonzentrierte Lösung können wie vorstehend erläutert wiederverwendet werden, was am zweckmäßigsten ist. Alternativ können Destillat, Lösungsmittel und Formamid bei ausreichender Qualität in das Verfahren zurückgeführt werden. Der Rückstand wird umkristallisiert, um Harnstoff und Katalysator voneinander zu trennen. Der Katalysator kann in dem Verfahren wiederverwendet werden.

### Beispiele

### Synthese von [Ru(Triphos)(tmm)]

Ein 35 mL Schlenk-Rohr wurde mit 319 mg (1,00 mmol) [Ru(cod)(methylallyl)] (cod = 1,5-Cyclooctadien) und 624 mg (1,17 mmol) 1,1,1-Tris(diphenylphosphinomethyl)ethan in 20 mL Toluol befüllt. Die Reaktionsmischung wurde gerührt und für 2 h auf 110°C erwärmt, auf Raumtemperatur abgekühlt und im Vakuum konzentriert. Nach Behandlung mit 15 mL Pentan wurde der ausfallende Komplex isoliert, mit Pentan gewaschen (3 x 10 mL) und über Nacht im Vakuum getrocknet, wodurch [Ru(Triphos)(tmm)] als hellgelbes Pulver erhalten wurde (0,531 g, 0,678 mmol, 68% Ausbeute). Die Identität wurde durch ¹H-, ¹³C-APT- und ³¹P-NMR-Spektren bestätigt.

### Beispiele 1-9

### Synthese von Harnstoff aus Formamid und Ammoniak mit Ru(Triphos)(tmm)

Die Synthese des Harnstoffs erfolgte gemäß folgender Reaktionsgleichung:

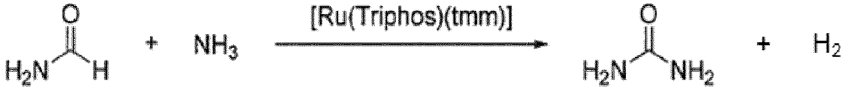

Es wurden Hochdruck-Chargenversuche in einem 10 mL Autoklaven ausgeführt, der mit einem Glaseinsatz und einem magnetischen Rührstab ausgerüstet war. Bei Verwendung von 2 mL 1,4-Dioxan sowie 0.6 g NH₃ betrug der Reaktionsdruck etwa 30 bar im warmen Zustand (Reaktionstemperatur) und der Druck im kalten Zustand (Raumtemperatur) etwa 8-10 bar. Vor dem Einsatz wurde der Autoklav mindestens 30 Minuten evakuiert und wiederholt mit Argon gefüllt. Der Katalysator [Ru(Triphos)(tmm)] (7,8 mg, 0,01 mmol) wurde unter ArgonAtmosphäre in ein Schlenk-Rohr eingewogen und in 1,4-Dioxan (2,0 mL) gelöst. Nach Zugabe von Formamid (40 µL, 1,00 mmol) wurde die Reaktionsmischung mit einer Kanüle unter Argon-Gegenstrom in den Autoklaven transferiert. Flüssiges NH₃ (zwischen 0,5 und 1,0 g) wurde in den Autoklaven gegeben und der Autoklav verschlossen. Die Reaktionsmischung wurde gerührt und in einem Aluminiumkegel für die jeweilige Reaktionszeit auf die jeweilige Reaktionstemperatur erwärmt. Nach Abkühlen auf Raumtemperatur wurde der Autoklav vorsichtig belüftet. Nach Entfernen des Lösungsmittels unter vermindertem Druck wurde die erhaltene Reaktionslösung durch ¹H- und ¹³C-NMR-Spektroskopie unter Verwendung von Mesitylen als internem Standard analysiert und die Ausbeute bestimmt.

Der Versuch wurde mehrmals wiederholt, wobei Katalysatorbeladung, Lösungsmittel, Reaktionstemperatur und Reaktionszeit wie in nachstehender Tabelle 1 gezeigt variiert wurden. In Tabelle 1 ist auch die erhaltene Ausbeute an Harnstoff gezeigt.

Die Katalysatorbeladung ist die Menge an eingesetzten Katalysator in mol-% bezogen auf die Menge an eingesetztem Formamid (in mol).

**Tabelle 1: Ru-katalysierte Synthese von Harnstoff aus Formamid und Ammoniak***

| Bsp. | Katalysatorbeladung [mol-%] | Lösungsmittel | Reaktionstemperatur [°C] | Reaktionszeit [Stunde] | Ausbeute [%] |
|---|---|---|---|---|---|
| 1 | 1,00 | 1,4-Dioxan | 150 | 5 | 44 |
| 2 | 1,00 | 1,4-Dioxan | 150 | 10 | 64 |
| 3 | 1,00 | 1,4-Dioxan | 150 | 15 | 57 |
| 4 | 1,00 | 1,4-Dioxan | 130 | 10 | 12 |
| 5 | 1,00 | 1,4-Dioxan | 110 | 10 | 1 |
| 6 | 0,50 | 1,4-Dioxan | 150 | 10 | 26 |
| 7 | 0,25 | 1,4-Dioxan | 150 | 10 | 14 |
| 8 | 1,00 | Toluol | 150 | 10 | 53 |
| 9 | 1,00 | THF | 150 | 10 | 47 |

| | | | | | |
|---|---|---|---|---|---|
| * Reaktionsbedingungen: [Ru(Triphos)(tmm)], 1 mmol Formamid, 2 mL Lösungsmittel, 0,5-1,0 g NH₃ | | | | | |

### Beispiel 10

### in situ-Herstellung von Ru(Triphos)(tmm) für Synthese von Harnstoff

Der Katalysator Ru(Triphos)(tmm) wurde in situ aus der Katalysatorvorstufe [Ru(cod)(methylallyl)₂] und Triphos gebildet.

Hierfür wurden 1 mol% [Ru(cod)(methylallyl)₂], 1,3 mol% Triphos, 1 mmol Formamid, 2 mL 1,4-Dioxan und 0,6 g NH₃ bei 150 °C für 10 h umgesetzt. Der Druck war etwa 8 bar im kalten Zustand und etwa 30 bar bei 150°C. Die Ausbeute an Harnstoff betrug 51%.

### Beispiel 11

### Synthese von Harnstoff aus Formamid in Abwesenheit von Ammoniak

1 mol% [Ru(Triphos)tmm], 1 mmol Formamid und 2 mL 1,4-Dioxan wurden bei 150 °C für 10 h bei 15 bar umgesetzt. Die Ausbeute an Harnstoff betrug 7%.

### Beispiele 12 bis 18

### Katalytische Aktivität von Ru-Phosphin-Komplexen in Abhängigkeit von den Liganden am Phosphor.

Die katalytische Aktivität von verschiedenen Ru-Phosphin-Komplexen bei der Synthese von Harnstoff aus Formamid und Ammoniak in Abhängigkeit von den Liganden am Phosphor wurde getestet. In Tabelle 2 sind die untersuchten Komplexe (Katalysatoren), die Reaktionsbedingungen und die erhaltenen Ausbeuten angeführt. Bei den Versuchen betrug der Reaktionsdruck etwa 30 bar bei der Reaktionstemperatur und der Druck im kalten Zustand etwa 8 bar, außer in Bsp. 15.

Es wurden Ruthenium-Triphosphin-Komplexe mit folgender Struktur untersucht:

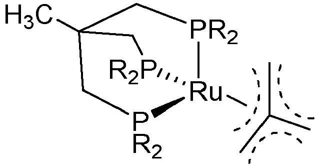

Die Art des Substituenten R ist in folgender Tabelle 2 wiedergegeben, wobei für den Fall, dass nicht alle Substituenten R an den drei Phosphoratomen gleich sind. die Substituenten R an einem ersten P-Atom als R¹, an einem zweiten P-Atom als R² und an einem dritten P-Atom als R³ bezeichnet werden. Beispielsweise hat der Komplex von Bsp. 16 an zwei Phosphingruppen jeweils zwei Phenylgruppen und die dritte Phosphingruppe weist zwei Isopropylgruppen auf.

Der Ruthenium-Triphosphin-Komplex verfügt außerdem über den dreizähnigen Liganden Trimethylenmethan.

Die in der Tabelle angegebenen Drücke beziehen sich auf die Raumtemperatur (etwa 23°C). Die Autoklaven wurden bei Raumtemperatur befüllt und dann auf Reaktionstemperatur und Reaktionsdruck gebracht.

**Tabelle 2**

| **Bsp.** | **R =** | | **Reaktionsbedingungen** | **Ausbeute Harnstoff** |
|---|---|---|---|---|
| 12 | 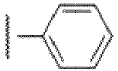 | | 1 mol% Kat., 1 mmol Formamid, 2 mL 1,4-Dioxan, 0,6 g NH₃, 150°C, 10 h | 64 % |
| 13 | 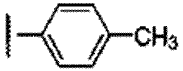 | | 0,5 mol% Kat., 1 mmol Formamid, 2 mL 1,4-Dioxan, 8 bar NH₃, 150°C, 20 h | 8% |
| 14 | 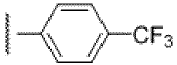 | | 0,5 mol% Kat., 1 mmol Formamid, 2 mL 1,4-Dioxan, 8 bar NH₃, 150°C, 20 h | 8% |
| 15 | 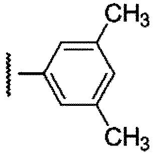 | | 1 mol% Kat., 2 mol% B(C₆F₅)₃, 1 mmol Formamid, 2 mL 1,4-Dioxan, 4 bar NH₃, 150°C, 20 h | 16% |
| 16 | 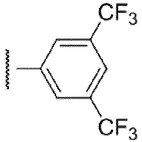 | | 0,5 mol% Kat., 1 mmol Formamid, 2 mL 1,4-Dioxan, 8 bar NH₃, 150°C, 20 h | 2% |
| 17 | R¹, R²: | R³: | 1 mol% Kat., 1 mmol Formamid, 2 mL 1,4-Dioxan, 8 bar NH₃, 150°C, 20 h | 30% |
| | 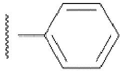 | 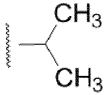 | | |
| 18 | | R³: | 0,2 mol% Kat., 5 mmol Formamid, 4,0 g NH₃, 150°C, 20 h | 6% |
| | 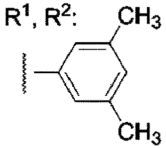 | 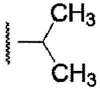 | | |

### Beispiele 19 bis 21

### Katalytische Aktivität von Ru-Phosphin-Komplexen in Abhängigkeit von den zusätzlichen Liganden an Ruthenium (Nicht-Phosphin-Liganden).

Die katalytische Aktivität von verschiedenen Ru-Phosphin-Komplexen bei der Synthese von Harnstoff aus Formamid und Ammoniak in Abhängigkeit von den Nicht-Phosphin-Liganden am Ruthenium wurde getestet. In Tabelle 3 sind die untersuchten Komplexe (Katalysatoren), die Reaktionsbedingungen und die erhaltenen Ausbeuten angeführt. Bei den Versuchen betrug der Druck etwa 30 bar bei der Reaktionstemperatur und etwa 8-10 bar im kalten Zustand (Raumtemperatur). Beispiel 19 entspricht Beispiel 12.

Es wurden Ruthenium-Triphosphin-Komplexe mit folgender Struktur untersucht:

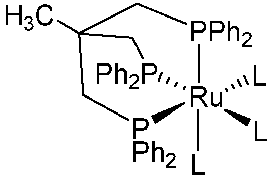

Die drei Liganden L sind in folgender Tabelle 3 wiedergegeben, wobei ein Ligand L als L¹, ein zweiter Ligand L als L² und ein dritter Ligand L als L³ bezeichnet wird. In Beispiel 19 werden die drei Liganden L zusammen durch den dreizähnigen Liganden Trimethylenmethan (tmm) gebildet. Die in der Tabelle angegebenen Drücke beziehen sich auf die Raumtemperatur (etwa 23°C). Die Autoklaven wurden bei Raumtemperatur befüllt und dann auf Reaktionstemperatur und Reaktionsdruck gebracht.

**Tabelle 3**

| **Bsp.** | **L =** | **Reaktionsbedingungen** | **Ausbeute Harnstoff** |
|---|---|---|---|
| 19 | 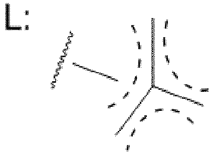 | 1 mol% Kat., 1 mmol Formamid, 2 mL 1,4-Dioxan, 0,6 g NH₃, 150°C, 10 h | 64 % |
| 20 | **L¹ = CO; L² = H; L³ = Cl** | 1 mol% Kat., 1 mmol Formamid, 2 mL 1,4-Dioxan, 8 bar NH₃, 150°C, 20 h | 18% |
| 21 | **L¹ = CO; L², L³ = H** | 1 mol% Kat., 1 mmol Formamid, 2 mL 1,4-Dioxan, 0,7 g NH₃, 150°C, 10 h | 24% |

### Beispiele 22 bis 28

### Katalytische Aktivität von Ru-Phosphin-Komplexen in Abhängigkeit von Katalysatorkonzentration

Die katalytische Aktivität in Abhängigkeit von der Katalysatorkonzentration wurde für folgende Reaktionsbedingungen getestet:
Katalysator: [Ru(Triphos)(tmm)], 1 mmol Formamid, 2 mL 1,4-Dioxan, 0,6 g NH₃, 150 °C, 10 h, wobei die Katalysatorkonzentration variiert wurde. Der Reaktionsdruck betrug etwa 30 bar bei der Reaktionstemperatur und der Druck im kalten Zustand etwa 8-10 bar.

In Tabelle 4 sind die unter diesen Reaktionsbedingungen eingesetzte Katalysatorkonzentration (in mol% bezogen auf Formamid) und die erhaltenen Ausbeuten angeführt.

**Tabelle 4**

| Bsp. | c(Kat.) [mol%] | Ausbeute Harnstoff [%] |
|---|---|---|
| 22 | 0,05 | < 1 |
| 23 | 0,25 | 16 |
| 24 | 1 | 40 |
| 25 | 5 | 63 |

Die katalytische Aktivität in Abhängigkeit von der Katalysatorkonzentration wurde ferner für folgende Reaktionsbedingungen getestet:
Katalysator: [Ru(Triphos)(tmm)], 1 mmol Formamid, 2 mL 1,4-Dioxan, 4 bar NH₃ bei Raumtemperatur (ca. 23°C), 150 °C, 20 h, wobei die Katalysatorkonzentration variiert wurde.

In Tabelle 5 sind die unter diesen Reaktionsbedingungen eingesetzte Katalysatorkonzentration (in mol% bezogen auf Formamid) und die erhaltenen Ausbeuten angeführt.

**Tabelle 5**

| Bsp. | c(Kat.) [mol%] | Ausbeute Harnstoff [%] |
|---|---|---|
| 26 | 0,25 | 25 |
| 27 | 0,5 | 30 |
| 28 | 2 | 33 |

### Beispiele 29 bis 35

### Katalytische Aktivität von Ru-Phosphin-Komplexen in Abhängigkeit von der Lösungsmittelkonzentration

Die katalytische Aktivität in Abhängigkeit von der Lösungsmittelkonzentration wurde für folgende Reaktionsbedingungen getestet:
Katalysator: 1 mol% [Ru(Triphos)(tmm)], 1 mmol Formamid, 0,6 g NH₃, 150 °C, 10 h, wobei die Lösungsmittelkonzentration variiert wurde. Der Reaktionsdruck betrug etwa 30 bar bei der Reaktionstemperatur und der Druck im kalten Zustand etwa 8-10 bar. Das Lösungsmittel war 1,4-Dioxan.

In Tabelle 6 sind die unter diesen Reaktionsbedingungen eingesetzte Menge an 1,4-Dioxan in ml (V(1,4-Dioxan) [mL]) und die erhaltenen Ausbeuten angeführt.

**Tabelle 6**

| Bsp. | V(1,4-Dioxan) [mL] | Ausbeute Harnstoff [%] |
|---|---|---|
| 29 | 0,5 | 53 |
| 30 | 0,8 | 45 |
| 31 | 1,1 | 50 |
| 32 | 1,4 | 60 |
| 33 | 1,7 | 42 |
| 34 | 2,3 | 37 |
| 35 | 2,6 | 31 |

### Beispiel 36

Ein für eine Ammoniaksynthese erzeugtes Synthesegas wird einer Gaswäsche unterzogen, bei der eine wässrige Ammoniak-Lösung mit einem Ammoniak-Anteil von etwa 5 bis 60 Gew.-%, bevorzugt etwa 5 bis 30 Gew.-%, für die Abtrennung des Kohlendioxids aus dem Synthesegas bei dem Druck der Synthesegaserzeugung von ca. 36 bar und einer Temperatur von etwa 40 °C bis 60 °C in einem Absorber eingesetzt wird. Die COz-Abtrennung aus Rohsynthesegas mit wässrigem Ammoniak ist ein gängiges Verfahren und wird z.B. auch in der US 2018/0282265 A1 beschrieben.

Die resultierende Lösung bzw. Suspension von chemisch und physikalisch gebundenem Kohlendioxid wird ohne weitere Aufarbeitung in einem separaten Reaktor mit einem Teilstrom des durch die Gaswäsche gereinigten Synthesegases oder einem Teilstrom des ungereinigten Synthesegases (Rohsynthesegas) als Wasserstoffquelle gemischt. Die Mischung erfolgt so, dass ein Verhältnis p(NH₃) : p(COz) : p(H₂) in der Mischung von 8:32:80 (p = Partialdruck bei Raumtemperatur (23 °C)) zumindest annähernd eingehalten wird.

Eine Katalysatorlösung aus dem Ruthenium-Phosphin-Komplex [Ru(Triphos)(tmm)] gelöst in Toluol mit einer Konzentration von 0,7 µmol Katalysator pro mL Toluol und 25 Äquivalenten Al(OTf)₃ bezogen auf die Stoffmenge des Katalysators oder Nafion als Säure werden zur Mischung zugesetzt. Die erhaltene Mischung bildet ein Zweiphasensystem (Wasser/Toluol) und wird bei einer Temperatur von etwa 100 °C und bei einem Druck von etwa 180 bar 12 h umgesetzt. Die TON bezogen auf Ammoniumformiat beträgt beispielsweise 6941.

Nach der Umsetzung wird die wässrige Phase abgetrennt. Die wässrige Phase wird dann einer thermischen Zersetzungsbehandlung bei 176°C und Umgebungsdruck unterworfen, wodurch das darin enthaltene Ammoniumformiat zu 83 % in Formamid überführt wird. Wasser wird während oder nach der thermischen Zersetzungsbehandlung abdestilliert. Anschließend kann das erhaltene Produkt Formamid zur Reinigung rektifiziert werden.

### Beispiel 37

Ein für eine Ammoniaksynthese erzeugtes Synthesegas wird einer Gaswäsche zur Abtrennung von COz unterzogen. Hierfür wird ein sogenanntes Rectisolverfahren, ein physikalisches Gasreinigungsverfahren mit Methanol als Waschmedium, für die Abtrennung des Kohlendioxids aus dem Synthesegas beim vorliegenden Druck der Synthesegaserzeugung von ca. 36 bar und einer Temperatur von etwa -40 °C bis -20 °C eingesetzt. Für die Absorbertemperatur wird dabei ein Wert zwischen -40 °C und -30 °C bevorzugt. Die resultierende Methanollösung von physikalisch gebundenem Kohlendioxid wird ohne weitere Aufarbeitung mit gegebenenfalls anschließender Kälteintegration und mit Hilfe einer Pumpe auf einen erhöhten Druck gebracht. Im Anschluss wird die beladene Methanollösung in einem separaten Reaktor mit einem Teilstrom des durch die Gaswäsche gereinigten Synthesegases oder einem Teilstrom des ungereinigten Synthesegases (Rohsynthesegas) als Wasserstoffquelle gemischt und mit dem in Methanol gelöstem Katalysator versetzt um die Reaktionssequenz gemäß Gl.4 und Gl. 5 durchzuführen. Für das Nachahmen des industriellen Prozesses wurden in einem 10 mL Autoklav bei Raumtemperatur der Ruthenium-Phosphin-Komplex [Ru(Triphos)(tmm)] (0,5 µmol, 0,001 mol% bezogen auf Methanol) und Aluminiumtriflat (Al(OTf)₃ (12,5 µmol, 0,025 mol% bezogen auf Methanol) als Lewis-Säure in 2 mL Methanol mit dem COz versetzt, so dass der Druck des Reaktionsgemisches bei ca. 40 bar (Druck der Synthesegaserzeugung) lag. Wasserstoff oder ein Wasserstoff-Stickstoff-Gemisch (molares Verhältnis3:1, das Nachahmen des Ammoniak-Synthesegases) wurde in den Autoklav geleitet, sodass der Gesamtdruck im Autoklav auf 120 bar gebracht wurde. Die Mischung wurde bei einer Temperatur von 100 °C und bei einem resultierenden Druck von etwa 180 bar 18 Stunden umgesetzt, wobei ein Ameisensäuremethylester enthaltendes Reaktionsgemisch gebildet wurde. Je nach Katalysatorbeladung wird eine turnover number (TON) für Ameisensäuremethylester von 4000 erreicht.

Das Reaktionsgemisch wurde abgekühlt, der Druck herabgesetzt und die gasförmigen Reaktanden entfernt. Das Ameisensäuremethylester enthaltende Gemisch wurde mit NH₃ versetzt (8 bar Gesamtdruck bei Raumtemperatur) und eine Stunde bei einer Temperatur von 60 °C gerührt (Reaktionsdruck von ca. 27 bar.) Anschließend kann das freigesetzte Methanol vom Produkt Formamid abdestilliert werden. Die Umsetzung von Ameisensäuremethylester mit Ammoniak zum Formamid verläuft nahezu quantitativ.

## Patentansprüche

1. Verfahren zur Herstellung von Harnstoff, umfassend:
a) Herstellen von Formamid auf Basis von Kohlendioxid, Wasserstoff und Ammoniak, wobei Methylformiat oder Ammoniumformiat als Zwischenprodukt in einer katalytischen Reaktion gebildet wird, und
b) Herstellen von Harnstoff durch Umsetzung des gebildeten Formamids oder des gebildeten Formamids mit Ammoniak in Anwesenheit eines Katalysators,
wobei die Quelle für Kohlendioxid eine mit chemisch und/oder physikalisch gebundenem Kohlendioxid beladene Flüssigkeit, ausgewählt aus einer Methanolphase oder einer wässrigen Ammoniaklösung, ist, die erhalten wird durch Gaswäsche eines Synthesegases zur Entfernung von COz unter Verwendung einer Waschflüssigkeit, wobei
a1) die Waschflüssigkeit eine Methanolphase ist oder COz aus der mit chemisch und/oder physikalisch gebundenem Kohlendioxid beladenen Waschflüssigkeit desorbiert und in eine Methanolphase absorbiert wird, um eine mit COz beladene Methanolphase zu erhalten, und
die mit COz beladene Methanolphase als COz enthaltender Strom mit einem Wasserstoff enthaltenden Strom in Anwesenheit eines Katalysators unter Bildung von Methylformiat umgesetzt wird und das gebildete Methylformiat mit einem Ammoniak enthaltenden Strom unter Bildung von Formamid umgesetzt wird, oder
a2) die Waschflüssigkeit eine wässrige Ammoniaklösung ist, so dass COz zumindest teilweise in Form von Carbonaten in der Waschflüssigkeit gebunden wird, und diese mit chemisch und/oder physikalisch gebundenem COz beladene Waschflüssigkeit als COz enthaltender Strom mit einem Wasserstoff enthaltenden Strom in Anwesenheit von einem Katalysator und gegebenenfalls organischem Lösungsmittel unter Bildung von Ammoniumformiat oder unter Bildung von Ammoniumformiat und Formamid umgesetzt wird und das gebildete Ammoniumformiat durch Wärmebehandlung in Formamid überführt wird.

2. Verfahren nach Anspruch 1, wobei das Synthesegas ein Synthesegas für die Ammoniaksynthese ist und/oder die Gaswäsche an einem aus der Dampfreformierung und/oder anschließender Wassergas-Shift-Reaktion erhaltenen Synthesegas ausgeführt wird.

3. Verfahren nach Anspruch 1 oder 2, wobei als Synthesegas ganz oder zumindest teilweise ein gegebenenfalls aufbereitetes Gas ausgewählt aus einem Koksofengas, einem Hochofengas, Konvertergas, oder einem Abgas von Zementwerken verwendet wird, gegebenenfalls in Mischung mit einem Synthesegas für die Ammoniaksynthese.

4. Verfahren nach einem der Ansprüche 1 bis 3, wobei für die Gaswäsche zur CO₂-Entfernung Methanol oder eine wässrige Ammoniaklösung als Waschflüssigkeit verwendet wird.

5. Verfahren nach einem der Ansprüche 1 bis 4, wobei
die Gaswäsche des Synthesegases zur Entfernung von COz aus dem Synthesegas mit der wässrigen Ammoniaklösung als Waschflüssigkeit bei einem Druck von 20 bis 50 bar und/oder bei einer Temperatur von unter 100 °C, bevorzugt im Bereich von 30 bis 70 °C, durchgeführt wird, oder die Gaswäsche des Synthesegases zur Entfernung von COz aus dem Synthesegas mit Methanol als Waschflüssigkeit bei einem Druck von 20 bis 50 bar und/oder mit einem auf eine Temperatur von -20 °C oder darunter abgekühlten Methanol durchgeführt wird.

6. Verfahren nach einem der Ansprüche 1 bis 5, wobei als Wasserstoff enthaltender Strom
ein Teilstrom des Synthesegases nach der Gaswäsche, wobei der Teilstrom vor oder nach einer gegebenenfalls durchgeführten Methanisierung des Synthesegases entnommen werden kann,
ein Teilstrom des Synthesegases vor der Gaswäsche,
ein aus der Aufarbeitung der Produkte der Ammoniak-Synthese und/oder
Harnstoff-Synthese erhaltener Wasserstoff oder eine Kombination davon verwendet wird.

7. Verfahren nach einem der Ansprüche 1 bis 6, wobei als Wasserstoff enthaltender Strom ein aus der Aufarbeitung der Produkte der Harnstoff-Synthese erhaltener Wasserstoff verwendet wird.

8. Verfahren nach einem der Ansprüche 1 bis 7, wobei als Ammoniak enthaltender Strom und/oder für die wässrige Ammoniaklösung Ammoniak verwendet wird, das aus dem Synthesegas in der Ammoniak-Synthese gewonnen wird, wobei gegebenenfalls zusätzlich aus der Aufarbeitung der Produkte der Harnstoff-Synthese erhaltenes Ammoniak für den Ammoniak enthaltenden Strom und/oder für die wässrige Ammoniaklösung verwendet wird.

9. Verfahren nach einem der Ansprüche 1 bis 8, wobei ein Ruthenium-Katalysator, insbesondere ein Ruthenium-Phosphin-Komplex, als Katalysator für die Umsetzung des gebildeten Formamids zur Bildung von Harnstoff oder für die Umsetzung des gebildeten Formamids mit Ammoniak zur Bildung von Harnstoff verwendet wird und/oder
für die Umsetzung der mit COz beladenen Methanolphase und dem Wasserstoff enthaltenden Strom zur Bildung von Methylformiat gemäß Variante a1) verwendet wird und/oder
für die Umsetzung der mit COz beladenen wässrigen Ammoniaklösung und dem Wasserstoff enthaltenden Strom zur Bildung von Ammoniumformiat oder zur Bildung von Ammoniumformiat und Formamid gemäß Variante a2) verwendet wird.

10. Verfahren nach Anspruch 9, wobei der Ruthenium-Phosphin-Komplex mindestens ein Monophosphin, ein Diphosphin, ein Triphosphin oder eine Verbindung mit mehr als drei Phosphingruppen aufweist, wobei das Phosphin die Formel PR¹R²R³ aufweist, worin R¹, R² und R³ unabhängig voneinander jeweils substituiertes oder unsubstituiertes Alkyl, substituiertes oder unsubstituiertes Aryl oder substituiertes oder unsubstituiertes Heteroaryl sind, wobei vorzugsweise R¹ Alkyl ist und R² und R³ unabhängig voneinander substituiertes oder unsubstituiertes Heteroaryl und/oder substituiertes oder unsubstituiertes Aryl, insbesondere Phenyl, sind.

11. Verfahren nach Anspruch 9 oder Anspruch 10, wobei der Ruthenium-Phosphin-Komplex ein Ruthenium-Triphosphin-Komplex ist, wobei das Triphosphin die allgemeine Formel I aufweist: 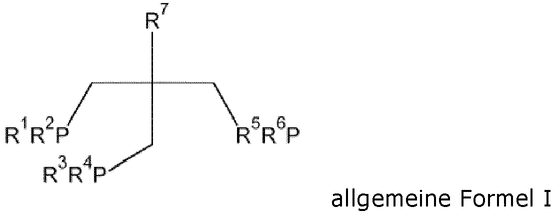 worin R¹ bis R⁶ unabhängig voneinander substituiertes oder unsubstituiertes Aryl oder substituiertes oder unsubstituiertes Heteroaryl, bevorzugt substituiertes oder unsubstituiertes Phenyl, sind und R⁷ Wasserstoff, Alkyl, Cycloalkyl oder Aryl ist, wobei das Triphosphin besonders bevorzugt 1,1,1-Tris(diphenylphosphinomethyl)ethan (Triphos) ist.

12. Verfahren nach irgendeinem der Ansprüche 9 bis 11, wobei der Ruthenium-Phosphin-Komplex die folgende allgemeine Formel II aufweist:
(A)Ru(L)₃ allgemeine Formel II
worin A ein Triphosphin der allgemeinen Formel I ist 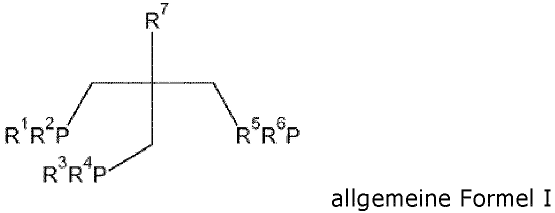 wobei R¹ bis R⁶ unabhängig voneinander substituiertes oder unsubstituiertes Aryl oder substituiertes oder unsubstituiertes Heteroaryl, bevorzugt substituiertes oder unsubstituiertes Phenyl, sind und R⁷ Wasserstoff, Alkyl, Cycloalkyl oder Aryl ist, und L jeweils unabhängig voneinander einzähnige Liganden sind, wobei zwei einzähnige Liganden L durch einen zweizähnigen Liganden ersetzt sein können oder drei einzähnige Liganden L durch einen dreizähnigen Liganden ersetzt sein können, und die ein-, zwei- oder dreizähnigen Liganden bevorzugt ausgewählt sind aus Trimethylenmethan (tmm), Cyclopentadienyl, Allyl, Methylallyl, Ethylen, Cyclooctadien, Acetylacetonat, Acetat, Hydrid, Halogenid, Phenolat, CO oder einer Kombination davon, wobei der Ruthenium-Phosphin-Komplex bevorzugt [Ru(Triphos)(tmm)] ist.

13. Verfahren nach einem der Ansprüche 1 bis 12, wobei für die Umsetzung der mit COz beladenen Methanolphase und dem Wasserstoff enthaltenden Strom zur Bildung von Methylformiat gemäß Variante a1) und/oder für die Umsetzung der mit COz beladenen wässrigen Ammoniaklösung und dem Wasserstoff enthaltenden Strom zur Bildung von Ammoniumformiat oder zur Bildung von Ammoniumformiat und Formamid gemäß Variante a2) ein Ruthenium-Phosphin-Komplex als Katalysator, gegebenenfalls in Kombination mit einer Säure, bevorzugt einer Lewis-Säure, als CoKatalysator, eingesetzt wird.

14. Verfahren nach einem der vorherigen Ansprüche, wobei die katalytische Umsetzung des gebildeten Formamids zur Bildung von Harnstoff oder die katalytische Umsetzung des gebildeten Formamids mit Ammoniak zur Bildung von Harnstoff bei einer Temperatur im Bereich von 50 bis 250 °C, bevorzugt im Bereich von 120 bis 200 °C und bevorzugter im Bereich von 140 bis 170 °C durchgeführt wird, und/oder bei einem Druck im Bereich von Umgebungsdruck bis 150 bar, bevorzugt im Bereich von 2 bar bis 60 bar, bevorzugter im Bereich von 5 bis 40 bar, durchgeführt wird.

15. Verfahren nach einem der vorherigen Ansprüche, wobei die katalytische Umsetzung des gebildeten Formamids zur Bildung von Harnstoff oder die katalytische Umsetzung des gebildeten Formamids mit Ammoniak zur Bildung von Harnstoff in einem unpolaren oder polaren aprotischen organischen Lösungsmittel, bevorzugt Toluol oder Dioxan, insbesondere 1,4-Dioxan, oder in flüssigem oder überkritischem Ammoniak durchgeführt wird.

16. Verfahren nach einem der vorherigen Ansprüche, wobei
die katalytische Umsetzung von der mit COz beladenen Methanolphase und dem Wasserstoff enthaltenden Strom zur Bildung von Methylformiat gemäß Variante a1) bei einer Temperatur im Bereich von 20 bis 150 °C, bevorzugt im Bereich von 60 bis 140°C, besonders bevorzugt im Bereich von 80 bis 120 °C und/oder bei einem Druck im Bereich von 40 bar bis 220 bar, bevorzugt im Bereich von 80 bar bis 200 bar, durchgeführt wird, und/oder wobei die Umsetzung des Methylformiats mit Ammoniak zum Formamid bei einer Temperatur im Bereich von 20 °C bis 100 °C, bevorzugt bei 60 bis 80 °C, und/oder bei einem Druck im Bereich von Atmosphärendruck bis 70 bar, bevorzugt im Bereich von Atmosphärendruck bis 45 bar durchgeführt wird, und/oder
wobei die katalytische Umsetzung von der mit chemisch gebundenem COz beladenen wässrigen Ammoniaklösung und dem Wasserstoff enthaltenden Strom zur Bildung von Ammoniumformiat oder zur Bildung von Ammoniumformiat und Formamid gemäß Variante a2) bei einer Temperatur im Bereich von 60 bis 180 °C, bevorzugt im Bereich von 90 bis 110 °C, und/oder bei einem Druck im Bereich von 35 bar bis 210 bar, bevorzugt im Bereich von 40 bar bis 195 bar, bevorzugter im Bereich von 80 bis 190 bar, durchgeführt wird, und/oder
wobei die Wärmebehandlung des Ammoniumformiats zur Bildung von Formamid bei einer Temperatur im Bereich von 100 °C bis 185 °C durchgeführt wird.

17. Verfahren nach einem der vorherigen Ansprüche, wobei das bei der Umsetzung von Methylformiat mit dem Ammoniak enthaltenden Strom zur Bildung von Formamid ebenfalls gebildete Methanol für die Waschflüssigkeit oder die Methanol enthaltende Flüssigkeit in dem Verfahren wiederverwendet wird.

18. Verfahren nach einem der vorherigen Ansprüche, wobei das Verfahren mit einer Ammoniak-Synthese gekoppelt ist, wobei die Ammoniaksynthese die Herstellung eines Synthesegases durch Dampfreformierung mit Wassergas-Shift-Reaktion, Gaswäsche des Synthesegases mit einer Waschflüssigkeit zur Entfernung von COz, Methanisierung des gereinigten Synthesegases und die Herstellung von Ammoniak mit dem gegebenenfalls verdichteten Synthesegas umfasst und für die Herstellung des Harnstoffs die mit COz beladene Waschflüssigkeit als Quelle für Kohlendioxid verwendet wird.

19. Verfahren nach Anspruch 18, wobei als Wasserstoff enthaltender Strom ein Teilstrom des Synthesegases nach der Gaswäsche, wobei der Teilstrom vor, während oder nach, bevorzugt vor, einer gegebenenfalls durchgeführten Methanisierung des Synthesegases entnommen werden kann, ein aus der Aufarbeitung der Produkte der Ammoniak-Synthese und/oder Harnstoff-Synthese erhaltener Wasserstoff oder eine Kombination davon verwendet wird und/oder
der bei der Ammoniaksynthese gebildete Ammoniak als Quelle für Ammoniak verwendet werden.

20. Verfahren nach Anspruch 18 oder 19, wobei das Verfahren für die Ammoniak-Synthese eine Dampfreformierung in einem Primärreformer und einen nachgeschalteten Sekundärreformer und/oder eine zweistufige Wassergas-Shift-Reaktion mit Hochtemperatur- und Niedertemperatur-Shiftstufe umfasst.

## Claims

1. A process for preparing urea, comprising:
a) preparing formamide on the basis of carbon dioxide, hydrogen and ammonia, with formation of methyl formate or ammonium formate as intermediate in a catalytic reaction, and
b) preparing urea by reacting the resultant formamide or the resultant formamide with ammonia in the presence of a catalyst,
where the source of carbon dioxide is a liquid laden with chemically and/or physically bound carbon dioxide and selected from a methanol phase or an aqueous ammonia solution which is obtained by gas scrubbing of a syngas for the removal of COz using a scrubbing fluid, where
a1) the scrubbing fluid is a methanol phase, or COz is desorbed from the scrubbing fluid laden with chemically and/or physically bound carbon dioxide and absorbed into a methanol phase to give a COz-laden methanol phase, and the COz-laden methanol phase is reacted as CO₂-containing stream with a hydrogen-containing stream in the presence of a catalyst to form methyl formate, and the resultant methyl formate is reacted with an ammonia-containing stream to form formamide, or
a2) the scrubbing fluid is an aqueous ammonia solution, and so COz is bound at least partly in the form of carbonates in the scrubbing fluid, and this scrubbing fluid laden with chemically and/or physically bound CO₂ is reacted as CO₂-containing stream with a hydrogen-containing stream in the presence of a catalyst and optionally organic solvent to form ammonium formate or to form ammonium formate and formamide, and the resultant ammonium formate is converted into formamide by heat treatment.

2. The process as claimed in claim 1, wherein the syngas is a syngas for the ammonia synthesis and/or the gas scrubbing is performed on a syngas obtained from steam reforming and/or subsequent water-gas shift reaction.

3. The process as claimed in claim 1 or 2, wherein syngas used comprises entirely or at least partly an optionally processed gas selected from a coke oven gas, a blast furnace gas, converter gas, or an offgas from cement works, optionally in a mixture with a syngas for the ammonia synthesis.

4. The process as claimed in any of claims 1 to 3, wherein methanol or an aqueous ammonia solution is used as scrubbing fluid for the gas scrubbing for COz removal.

5. The process as claimed in any of claims 1 to 4, wherein
the gas scrubbing of the syngas for the removal of COz from the syngas is carried out with the aqueous ammonia solution as scrubbing fluid at a pressure of 20 to 50 bar and/or at a temperature of below 100°C, preferably in the range from 30 to 70°C, or
the gas scrubbing of the syngas for the removal of COz from the syngas is carried out with methanol as scrubbing fluid at a pressure of 20 to 50 bar and/or with a methanol cooled to a temperature of -20°C or below.

6. The process as claimed in any of claims 1 to 5, wherein the hydrogen-containing stream used comprises
a substream of the syngas after the gas scrubbing, where the substream may be withdrawn before or after a syngas methanization carried out optionally,
a substream of the syngas before the gas scrubbing,
a hydrogen obtained from the processing of the products of the ammonia synthesis and/or urea synthesis, or a combination thereof.

7. The process as claimed in any of claims 1 to 6, wherein the hydrogen-containing stream used is a hydrogen obtained from the processing of the products of the urea synthesis.

8. The process as claimed in any of claims 1 to 7, wherein ammonia used as ammonia-containing stream and/or for the aqueous ammonia solution is ammonia obtained from the syngas in the ammonia synthesis, where optionally ammonia obtained from the processing of the products of the urea synthesis is additionally used for the ammonia-containing stream and/or for the aqueous ammonia solution.

9. The process as claimed in any of claims 1 to 8, wherein a ruthenium catalyst, more particularly a ruthenium-phosphine complex, is used as catalyst
for the reaction of the resultant formamide to form urea or for the reaction of the resultant formamide with ammonia to form urea, and/or
for the reaction of the CO₂-laden methanol phase and the hydrogen-containing stream to form methyl formate in variant a1), and/or
for the reaction of the CO₂-laden aqueous ammonia solution and the hydrogen-containing stream to form ammonium formate or to form ammonium formate and formamide in variant a2).

10. The process as claimed in claim 9, wherein the ruthenium-phosphine complex comprises at least one monophosphine, one diphosphine, one triphosphine or one compound having more than three phosphine groups, the phosphine having the formula PR¹R²R³, in which R¹, R² and R³ independently of one another are in each case substituted or unsubstituted alkyl, substituted or unsubstituted aryl or substituted or unsubstituted heteroaryl, where preferably R¹ is alkyl and R² and R³ independently of one another are substituted or unsubstituted heteroaryl and/or substituted or unsubstituted aryl, more particularly phenyl.

11. The process as claimed in claim 9 or claim 10, wherein the ruthenium-phosphine complex is a ruthenium-triphosphine complex, the triphosphine having the general formula I: 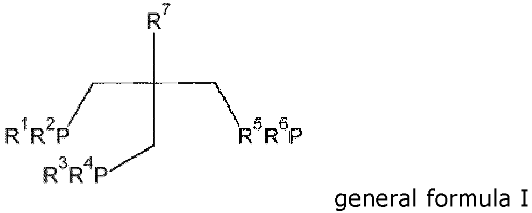 in which R¹ to R⁶ independently of one another are substituted or unsubstituted aryl or substituted or unsubstituted heteroaryl, preferably substituted or unsubstituted phenyl, and R⁷ is hydrogen, alkyl, cycloalkyl or aryl, the triphosphine being more preferably 1,1,1-tris(diphenylphosphino-methyl)ethane (triphos).

12. The process as claimed in any one of claims 9 to 11, wherein the ruthenium-phosphine complex has the following general formula II:
(A)Ru(L)₃ general formula II
in which A is a triphosphine of the general formula I 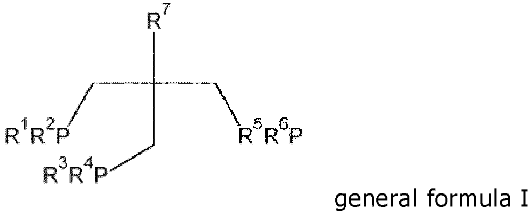 where R¹ to R⁶ independently of one another are substituted or unsubstituted aryl or substituted or unsubstituted heteroaryl, preferably substituted or unsubstituted phenyl, and R⁷ is hydrogen, alkyl, cycloalkyl or aryl, and L in each case independently of one another are monodentate ligands, it being possible for two monodentate ligands L to be replaced by one bidentate ligand or for three monodentate ligands L to be replaced by one tridentate ligand, and the mono-, di- or tridentate ligands are preferably selected from trimethylenemethane (tmm), cyclopentadienyl, allyl, methylallyl, ethylene, cyclooctadiene, acetylacetonate, acetate, hydride, halide, phenoxide, CO or a combination thereof, the ruthenium-phosphine complex preferably being [Ru(triphos)(tmm)].

13. The process as claimed in any of claims 1 to 12, wherein the reaction of the COz-laden methanol phase and the hydrogen-containing stream to form methyl formate in variant a1) and/or the reaction of the CO₂-laden aqueous ammonia solution and the hydrogen-containing stream to form ammonium formate or to form ammonium formate and formamide in variant a2) are/is carried out using a ruthenium-phosphine complex as catalyst, optionally in combination with an acid, preferably a Lewis acid, as cocatalyst.

14. The process as claimed in any of the preceding claims, wherein the catalytic reaction of the resultant formamide to form urea or the catalytic reaction of the resultant formamide with ammonia to form urea is carried out at a temperature in the range from 50 to 250°C, preferably in the range from 120 to 200°C and more preferably in the range from 140 to 170°C, and/or at a pressure in the range from ambient pressure to 150 bar, preferably in the range from 2 bar to 60 bar, more preferably in the range from 5 to 40 bar.

15. The process as claimed in any of the preceding claims, wherein the catalytic reaction of the resultant formamide to form urea or the catalytic reaction of the resultant formamide with ammonia to form urea is carried out in a nonpolar or polar aprotic organic solvent, preferably toluene or dioxane, more particularly 1,4-dioxane, or in liquid or supercritical ammonia.

16. The process as claimed in any of the preceding claims, wherein
the catalytic reaction of the CO₂-laden methanol phase and the hydrogen-containing stream to form methyl formate in variant a1) is carried out at a temperature in the range from 20 to 150°C, preferably in the range from 60 to 140°C, more preferably in the range from 80 to 120°C and/or at a pressure in the range from 40 bar to 220 bar, preferably in the range from 80 bar to 200 bar, and/or
the reaction of the methyl formate with ammonia to formamide is carried out at a temperature in the range from 20°C to 100°C, preferably at 60 to 80°C, and/or at a pressure in the range from atmospheric pressure to 70 bar, preferably in the range from atmospheric pressure to 45 bar, and/or the catalytic reaction of the aqueous ammonia solution laden with chemically bound CO₂ and the hydrogen-containing stream to form ammonium formate or to form ammonium formate and formamide in variant a2) is carried out at a temperature in the range from 60 to 180°C,
preferably in the range from 90 to 110°C, and/or at a pressure in the range from 35 bar to 210 bar, preferably in the range from 40 bar to 195 bar, more preferably in the range from 80 to 190 bar, and/or
the heat treatment of the ammonium formate to form formamide is carried out at a temperature in the range from 100°C to 185°C.

17. The process as claimed in any of the preceding claims, wherein the methanol likewise formed in the reaction of methyl formate with the ammonia-containing stream to form formamide is reused for the scrubbing fluid or the methanol-containing liquid in the process.

18. The process as claimed in any of the preceding claims, wherein the process is coupled with an ammonia synthesis, the ammonia synthesis comprising the preparation of a syngas by steam reforming with water-gas shift reaction, gas scrubbing of the syngas with a scrubbing fluid for the removal of CO₂, methanization of the purified syngas, and the preparation of ammonia with the optionally compressed syngas, and the CO₂-laden scrubbing fluid is used as a source of carbon dioxide for the preparation of the urea.

19. The process as claimed in claim 18, wherein the hydrogen-containing stream used comprises a substream of the syngas after the scrubbing, it being possible for the substream to be withdrawn before, during or after, preferably before, a syngas methanization carried out optionally, a hydrogen obtained from the processing of the products of the ammonia synthesis and/or urea synthesis, or a combination thereof, and/or
the source of ammonia used comprises ammonia formed in the ammonia synthesis.

20. The process as claimed in claim 18 or 19, wherein the process for the ammonia synthesis comprises a steam reforming in a primary reformer and a downstream secondary reformer and/or a two-stage water-gas shift reaction with high-temperature shift stage and low-temperature shift stage.

## Revendications

1. Procédé de préparation d'urée, comprenant :
a) la préparation de formamide à base de dioxyde de carbone, d'hydrogène et d'ammoniac, du formiate de méthyle ou du formiate d'ammonium étant formé en tant que produit intermédiaire dans une réaction catalytique, et
b) la préparation d'urée par transformation du formamide formé ou du formamide formé avec de l'ammoniac en présence d'un catalyseur,
la source de dioxyde de carbone étant un liquide chargé de dioxyde de carbone lié chimiquement et/ou physiquement, choisi parmi une phase au méthanol ou une solution aqueuse d'ammoniac, qui est obtenu par lavage de gaz d'un gaz de synthèse pour l'élimination de CO₂ en utilisant un liquide de lavage,
a1) le liquide de lavage étant une phase au méthanol ou du CO₂ étant désorbé du liquide de lavage chargé de dioxyde de carbone lié chimiquement et/ou physiquement et étant absorbé dans une phase au méthanol, afin d'obtenir une phase au méthanol chargée de CO₂, et la phase au méthanol chargée de CO₂, en tant que flux contenant du CO₂, étant transformée avec un flux contenant de l'hydrogène en présence d'un catalyseur avec formation de formiate de méthyle et le formiate de méthyle formé étant transformé avec un flux contenant de l'ammoniac avec formation de formamide, ou
a2) le liquide de lavage étant une solution aqueuse d'ammoniac, de sorte que du CO₂ soit lié au moins partiellement sous forme de carbonates dans le liquide de lavage, et celui-ci étant transformé avec un liquide de lavage chargé de CO₂ lié chimiquement et/ou physiquement, en tant que flux contenant du CO₂, avec un flux contenant de l'hydrogène en présence d'un catalyseur et éventuellement d'un solvant organique avec formation de formiate d'ammonium ou avec formation de formiate d'ammonium et de formamide et le formiate d'ammonium formé étant converti en formamide par traitement thermique.

2. Procédé selon la revendication 1, le gaz de synthèse étant un gaz de synthèse pour la synthèse d'ammoniac et/ou le lavage de gaz étant réalisé sur un gaz de synthèse obtenu à partir du reformage à la vapeur et/ou de la réaction de déplacement de gaz à l'eau consécutive.

3. Procédé selon la revendication 1 ou 2, dans lequel, en tant que gaz de synthèse, un gaz éventuellement préparé, choisi parmi un gaz de four à coke, un gaz de haut fourneau, un gaz de convertisseur ou un gaz d'échappement de cimenterie, est utilisé entièrement ou au moins en partie, éventuellement en mélange avec un gaz de synthèse pour la synthèse de l'ammoniac.

4. Procédé selon l'une quelconque des revendications 1 à 3, dans lequel, pour le lavage de gaz pour l'élimination de CO₂, du méthanol ou une solution aqueuse d'ammoniac est utilisé(e) en tant que liquide de lavage.

5. Procédé selon l'une quelconque des revendications 1 à 4, le lavage de gaz du gaz de synthèse, pour éliminer du CO₂ du gaz de synthèse, étant réalisé avec la solution aqueuse d'ammoniac en tant que liquide de lavage à une pression de 20 à 50 bars et/ou à une température inférieure à 100 °C, préférablement dans la plage de 30 à 70 °C, ou le lavage de gaz du gaz de synthèse, pour éliminer du CO₂ du gaz de synthèse, étant réalisé avec du méthanol en tant que liquide de lavage à une pression de 20 à 50 bars et/ou avec un méthanol refroidi à une température de -20 °C ou en dessous.

6. Procédé selon l'une quelconque des revendications 1 à 5, dans lequel, en tant que flux contenant de l'hydrogène,
un flux partiel du gaz de synthèse après le lavage de gaz, le flux partiel pouvant être prélevé avant ou après une méthanation du gaz de synthèse éventuellement réalisée,
un flux partiel du gaz de synthèse avant le lavage de gaz,
un hydrogène obtenu à partir du traitement des produits de la synthèse d'ammoniac et/ou de la synthèse d'urée, ou une combinaison correspondante étant utilisé(e).

7. Procédé selon l'une quelconque des revendications 1 à 6, dans lequel, en tant que flux contenant de l'hydrogène, un hydrogène obtenu à partir du traitement des produits de la synthèse d'urée est utilisé.

8. Procédé selon l'une quelconque des revendications 1 à 7, dans lequel, en tant que flux contenant de l'ammoniac et/ou pour la solution aqueuse d'ammoniac, de l'ammoniac est utilisé qui est produit à partir du gaz de synthèse dans la synthèse d'ammoniac, dans lequel, éventuellement en plus, de l'ammoniac obtenu à partir du traitement des produits de la synthèse d'urée est utilisé pour le flux contenant de l'ammoniac et/ou pour la solution aqueuse d'ammoniac

9. Procédé selon l'une quelconque des revendications 1 à 8, dans lequel un catalyseur au ruthénium, en particulier un complexe ruthénium-phosphine, est utilisé en tant que catalyseur pour la transformation du formamide formé pour la formation d'urée ou pour la transformation du formamide formé avec de l'ammoniac pour la formation d'urée et/ou est utilisé pour la transformation de la phase au méthanol chargée de CO₂ et du flux contenant de l'hydrogène pour la formation de formiate de méthyle selon la variante a1) et/ou
est utilisé pour la transformation de la solution aqueuse d'ammoniac chargée de CO₂ et du flux contenant de l'hydrogène pour la formation de formiate d'ammonium ou pour la formation de formiate d'ammonium et de formamide selon la variante a2).

10. Procédé selon la revendication 9, le complexe ruthénium-phosphine présentant au moins une monophosphine, une diphosphine, une triphosphine ou un composé comportant plus de trois groupes phosphine, la phosphine présentant la formule PR¹R²R³, dans laquelle R¹, R² et R³ sont indépendamment les uns des autres à chaque fois alkyle substitué ou non substitué, aryle substitué ou non substitué ou hétéroaryle substitué ou non substitué, de préférence R¹ étant alkyle et R² et R³ indépendamment l'un de l'autre étant hétéroaryle substitué ou non substitué et/ou aryle substitué ou non substitué, en particulier phényle.

11. Procédé selon la revendication 9 ou la revendication 10, le complexe ruthénium-phosphine étant un complexe ruthénium-triphosphine, la triphosphine présentant la formule générale I : 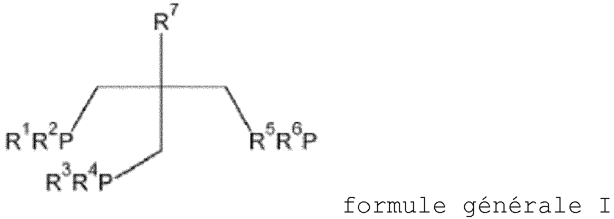 dans laquelle R¹ à R⁶ sont indépendamment les uns des autres aryle substitué ou non substitué ou hétéroaryle substitué ou non substitué, préférablement phényle substitué ou non substitué, et R⁷ est hydrogène, alkyle, cycloalkyle ou aryle, la triphosphine étant particulièrement préférablement le 1,1,1-tris(diphénylphosphinométhyl)éthane (Triphos).

12. Procédé selon l'une quelconque des revendications 9 à 11, le complexe ruthénium-phosphine présentant la formule générale suivante II :
(A)Ru(L)₃ formule générale II
dans laquelle A est triphosphine de formule générale I 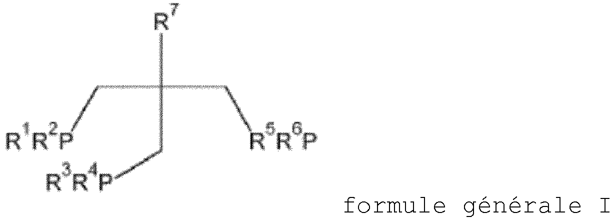 dans laquelle R¹ à R⁶ sont indépendamment les uns des autres aryle substitué ou non substitué ou hétéroaryle substitué ou non substitué, préférablement phényle substitué ou non substitué, et R⁷ est hydrogène, alkyle, cycloalkyle ou aryle, et L sont à chaque fois indépendamment les uns des autres des ligands monodentates, deux ligands monodentates L pouvant être remplacés par un ligand bidentate ou trois ligands monodentates L pouvant être remplacés par un ligand tridentate, et les ligands monodentates, bidentates et tridentates étant préférablement choisis parmi triméthylèneméthane (tmm), cyclopentadiényle, allyle, méthylallyle, éthylène, cyclooctadiène, acétylacétonate, acétate, hydrure, halogénure, phénolate, CO ou une combinaison correspondante, le complexe ruthénium-phosphine étant préférablement [Ru (Triphos) (tmm)].

13. Procédé selon l'une quelconque des revendications 1 à 12, dans lequel pour la transformation de la phase au méthanol chargée de CO₂ et du flux contenant de l'hydrogène pour la formation de formiate de méthyle selon la variante a1) et/ou pour la transformation de la solution aqueuse d'ammoniac chargée de CO₂ et du flux contenant de l'hydrogène pour la formation de formiate d'ammonium ou pour la formation de formiate d'ammonium et de formamide selon la variante a2), un complexe ruthénium-phosphine est utilisé en tant que catalyseur, éventuellement en combinaison avec un acide, préférablement un acide de Lewis, en tant que co-catalyseur.

14. Procédé selon l'une quelconque des revendications précédentes, la transformation catalytique du formamide formé pour la formation d'urée ou la transformation catalytique du formamide formé avec de l'ammoniac pour la formation d'urée est réalisée à une température dans la plage de 50 à 250 °C, préférablement dans la plage de 120 à 200 °C et plus préférablement dans la plage de 140 à 170 °C, et/ou à une pression dans la plage de la pression ambiante jusqu'à 150 bars, préférablement dans la plage de 2 bars à 60 bars, plus préférablement dans la plage de 5 à 40 bars.

15. Procédé selon l'une quelconque des revendications précédentes, la transformation catalytique du formamide formé pour la formation d'urée ou la transformation catalytique du formamide formé avec de l'ammoniac pour la formation d'urée est réalisée dans un solvant organique aprotique apolaire ou polaire, préférablement le toluène ou le dioxanne, en particulier le 1,4-dioxanne, ou dans de l'ammoniac liquide ou supercritique.

16. Procédé selon l'une quelconque des revendications précédentes, la transformation catalytique de la phase au méthanol chargée de CO₂ et du flux contenant de l'hydrogène pour la formation de formiate de méthyle selon la variante a1) étant réalisée à une température dans la plage de 20 à 150 °C, préférablement dans la plage de 60 à 140 °C, particulièrement préférablement dans la plage de 80 à 120 °C et/ou à une pression dans la plage de 40 bars à 220 bars, préférablement dans la plage de 80 bars à 200 bars, et/ou la transformation du formiate de méthyle avec de l'ammoniac en formamide étant réalisée à une température dans la plage de 20 °C à 100 °C, préférablement à une température de 60 à 80 °C, et/ou à une pression dans la plage de la pression atmosphérique jusqu'à 70 bars, préférablement dans la plage de la pression atmosphérique jusqu'à 45 bars, et/ou
la transformation catalytique de la solution aqueuse d'ammoniac chargée de CO₂ lié chimiquement et du flux contenant de l'hydrogène pour la formation de formiate d'ammonium ou pour la formation de formiate d'ammonium et de formamide selon la variante a2) étant réalisée à une température dans la plage de 60 à 180 °C, préférablement dans la plage de 90 à 110 °C et/ou à une pression dans la plage de 35 bars à 210 bars, préférablement dans la plage de 40 bars à 195 bars, plus préférablement dans la plage de 80 à 190 bars, et/ou
le traitement thermique du formiate d'ammonium pour la formation de formamide étant réalisé à une température dans la plage de 100 °C à 185 °C.

17. Procédé selon l'une quelconque des revendications précédentes, le méthanol également formé lors de la transformation de formiate de méthyle avec le flux contenant de l'ammoniac pour la formation de formamide étant réutilisé dans le procédé pour le liquide de lavage ou le liquide contenant du méthanol.

18. Procédé selon l'une quelconque des revendications précédentes, le procédé étant couplé à une synthèse d'ammoniac, la synthèse d'ammoniac comprenant la préparation d'un gaz de synthèse par reformage à la vapeur avec une réaction de déplacement de gaz à l'eau, par lavage de gaz du gaz de synthèse avec un liquide de lavage pour l'élimination de CO₂, par méthanation du gaz de synthèse purifié et la préparation d'ammoniac avec le gaz de synthèse éventuellement densifié et, pour la préparation de l'urée, le liquide de lavage chargé de CO₂ étant utilisé en tant que source de dioxyde de carbone.

19. Procédé selon la revendication 18, dans lequel, en tant que flux contenant de l'hydrogène, un flux partiel du gaz de synthèse après le lavage de gaz, le flux partiel pouvant être prélevé avant, pendant ou après, préférablement avant, une méthanation du gaz de synthèse éventuellement réalisée, un hydrogène obtenu à partir du traitement des produits de la synthèse d'ammoniac et/ou de la synthèse d'urée ou une combinaison correspondante étant utilisé(e) et/ou
l'ammoniac formé lors de la synthèse d'ammoniac étant utilisé en tant que source d'ammoniac.

20. Procédé selon la revendication 18 ou 19, le procédé pour la synthèse d'ammoniac comprenant un reformage à la vapeur dans un reformeur primaire et un reformeur secondaire en aval et/ou une réaction de déplacement de gaz à l'eau en deux étapes comportant une étape de déplacement à température élevée et à basse température.
